# EUROPEAN PATENT SPECIFICATION

(11) **EP 3 519 564 B1**
(45) Date of publication and mention of the grant of the patent: **15.11.2023**
(21) Application number: 17855171.9
(22) Date of filing: 28.09.2017
(51) Int. Cl.: C12N 5/10, A61P 35/00, A61K 35/17, C07K 14/705, A61K 35/00, A61K 38/00, A61K 39/00, C07K 14/725, C07K 16/28, C12N 5/0783, C12Q 1/68

(54) **A UNIVERSAL PLATFORM FOR CAR THERAPY TARGETING A NOVEL ANTIGENIC SIGNATURE OF CANCER**
UNIVERSELLE PLATTFORM FÜR CAR-THERAPIE GEGEN EINE NEUARTIGE ANTIGENSIGNATUR VON KREBS
PLATE-FORME UNIVERSELLE POUR THÉRAPIE CAR CIBLANT UNE NOUVELLE SIGNATURE ANTIGÉNIQUE DU CANCER

(30) Priority: 28.09.2016 US 201662400737 P
(43) Date of publication of application: 07.08.2019
(60) Divisional application: 23203075.9
(73) Proprietor: Gavish-Galilee Bio Applications Ltd, 1101602 Kiryat Shmona (IL); Immpact-Bio Ltd., Ness-Ziona 7403635 (IL)
(72) Inventor: GROSS, Gideon, 1292200 Moshav Almagor (IL); BEIMAN, Merav, Ness Ziona 7420827 (IL); GIBSON, William, Boston MA 02116 (US); DAHARY, Dvir, Ness Ziona 7403635 (IL)
(74) Representative: Dolphin, Kirsty Mairi
(86) International application number: PCT/IL2017/051102
(87) International publication number: WO 2018/061012

(56) References cited:
- WO-A1-2015/142314
- LEONARDO CHICAYBAM ET AL: "Abstract 2797: Construction and validation of an activating and inhibitory chimeric antigen receptor (CAR) system", CANCER RESEARCH, vol. 74, no. 19 Supplement, 1 October 2014 (2014-10-01), pages 2797-2797, XP055210536, ISSN: 0008-5472, DOI: 10.1158/1538-7445.AM2014-2797
- Leonardo Chicaybam ET AL: "Abstract 3156: Construction and validation of an activating and inhibitory chimeric antigen receptor (CAR) system", Cancer Research, 1 August 2015 (2015-08-01), XP055677331, Retrieved from the Internet: URL:https://cancerres.aacrjournals.org/con tent/75/15_Supplement/3156 [retrieved on 2020-03-18]
- JAINA M PATEL ET AL: "Cancer CARtography: charting out a new approach to cancer immunotherapy", IMMUNOTHERAPY, vol. 6, no. 6, 1 June 2014 (2014-06-01), pages 675-678, XP055188250, GB ISSN: 1750-743X, DOI: 10.2217/imt.14.44
- FEDOROV, VICTOR D. ET AL.: 'PD-1-and CTLA-4-based inhibitory chimeric antigen receptors (iCARs) divert off-target immunotherapy responses' SCIENCE TRANSLATIONAL MEDICINE, [Online] vol. 5, no. 215, 11 December 2013, XP055210508 Retrieved from the Internet: <URL:https://www.ncbi.nlm.nih.gov/pmc/artic les/PMC4238416>

## Description

### FIELD OF THE INVENTION:

The invention relates to the field of cancer immunotherapy by adoptive cell transfer, employing activating chimeric antigen receptors (aCARs) recognizing antigens expressed on the surface of tumor cells, and inhibitory CARs (iCARs) directed at allelic variants of the same or other cell surface antigens expressed by normal cells but not by the tumor due to loss of heterozygosity (LOH).

### BACKGROUND

The identification of targetable antigens that are exclusively expressed by tumor cells but not by healthy tissue is undoubtedly the major challenge in cancer immunotherapy today. Clinical evidence that T cells are capable of eradicating tumor cells comes from numerous studies evaluating highly diverse approaches for harnessing T cells to treat cancer (Rosenberg and Restifo, 2015). These employ bone marrow transplantation with donor lymphocyte infusion, adoptive transfer of tumor-infiltrating lymphocytes (TILs), treatment with T cells genetically redirected at pre-selected antigens via CARs (Gross and Eshhar, 2016a) or T cell receptors (TCRs), the use of immune checkpoint inhibitors or active vaccination. Of these, the use of genetically engineered T cells and different strategies for active immunization entail pre-existing information on candidate antigens which are likely to exert a durable clinical response but minimal adverse effects. Yet, as stated in the title of a recent review by S. Rosenberg, "Finding suitable targets is the major obstacle to cancer gene therapy" (Rosenberg, 2014).

The concept of using chimeric antigen receptors (or CARs) to genetically redirect T cells (or other killer cells of the immune system such as natural killer (NK) cells and cytokine-induced killer cells) against antigens of choice in an MHC-independent manner was first introduced by Gross and Eshhar in the late 1980s (Gross et al., 1989). They are produced synthetically from chimeric genes encoding an extracellular single-chain antibody variable fragment (scFv) fused through a flexible hinge and transmembrane canonic motif to signaling components comprising immunoreceptor tyrosine-based activation motifs of CD3-ζ or FcRγ chains capable of T cell activation. At present, CARs are being examined in dozens of clinical trials and have so far shown exceptionally high efficacy in B cell malignancies (Dotti et al., 2014; Gill and June, 2015; Gross and Eshhar, 2016a). The safety of CAR-T cell therapy is determined, in large, by its ability to discriminate between the tumor and healthy tissue. A major risk and the direct cause for adverse autoimmune effects that have been reported in clinical and preclinical studies is off-tumor, on-target toxicity resulting from extra-tumor expression of the target antigen (dealt with in detail in our recent review (Gross and Eshhar, 2016b) and (Klebanoff et al., 2016)). Concerning this risk, shared, non-mutated cell surface antigens which are currently tested clinically or pre-clinically for CAR therapy can be generally divided into a number of categories according to their tissue distribution and mode of expression:
- Strictly tumor-specific antigens. Perhaps the only member in this group which is already being examined clinically is variant III of the epidermal growth factor receptor (EGFRvIII) that is frequently overexpressed in glioblastoma and is also found in non-small cell lung carcinoma and prostate, breast, head and neck and ovarian cancers but not on normal tissue.
- Surface antigens expressed on the tumor and on non-vital healthy tissue. Potential CAR antigens in this group are differentiation-related molecules that are mainly restricted to the B cell lineage. Prominent among these (and a target antigen in numerous clinical trials) is CD19, a pan-B cell marker acquired very early in B cell differentiation and involved in signal transduction by the B cell receptor (BCR). Membrane prostate antigens constitute another class of antigens in this category.
- Antigens that are typically expressed by non-malignant tumor-promoting cells. One such antigen is fibroblast activation protein (FAP), a cell surface serine protease which is almost invariably expressed by tumor-associated fibroblasts in diverse primary and metastatic cancers. Another antigen is vascular endothelial growth factor (VEGF), which is highly expressed during tumor angiogenesis and is normally expressed on vascular and lymphatic endothelial cells in many vital organs.
- Tumor associated antigens (TAAs) shared with vital healthy tissue.

Most other TAAs which are presently evaluated in preclinical and clinical studies are overexpressed by tumors but are also present, usually at lower level, on essential normal tissue.

The broad spectrum of strategies devised to tackle autoimmunity in CAR T cell therapy can be divided into those which seek to eliminate, or suppress transferred T cells once damage is LOH, but that is expressed by the cells of the normal tissue, thereby identifying a personalized biomarker for the subject.

In still a further aspect, the present invention is directed to a safe effector immune cell as defined herein for use in treating a patient having a tumor characterized by LOH, wherein the iCAR is directed to a single allelic variant encoding a polymorphic cell surface epitope absent from cells of the tumor due to loss of heterozygosity (LOH) but present at least on all cells of related mammalian normal tissue of the patient.

The description describes a method for treating cancer in a patient having a tumor characterized by LOH comprising: (i) identifying or receiving information identifying a single allelic variant of a polymorphic cell surface epitope that is not expressed by cells of the tumor due to LOH, but that is expressed by the cells of the normal tissue, (ii) identifying or receiving information identifying a non-polymorphic cell surface epitope of an antigen or a single allelic variant of a polymorphic cell surface epitope, wherein said epitope is a tumor-associated antigen or is shared by cells at least of related tumor and normal tissue in said cancer patient; (iii) selecting or receiving at least one nucleic acid molecule defining an iCAR as defined herein and at least one nucleic acid molecule comprising a nucleotide sequence encoding an aCAR as defined herein, or at least one vector as defined herein, wherein the iCAR comprises an extracellular domain that specifically binds to a cell surface epitope of (i) and the aCAR comprises an extracellular domain that specifically binds to a cell surface epitope of (ii); (iv) preparing or receiving at least one population of safe redirected effector immune cells by transfecting effector immune cells with the nucleic acid molecules of (iii) or transducing effector immune cells with the vectors of (iii); and (v) administering to said cancer patient at least one population of safe redirected immune effector cells of (iv).

The description describes at least one population of safe redirected immune effector cells for treating cancer in a patient having a tumor characterized by LOH, wherein the safe redirected immune cells are obtained by (i) identifying or receiving element functions as a 'genetic switch' which exploits the mode of action of several cell surface receptors, including Notch. Following binding of such a receptor to its ligand it undergoes dual cleavage resulting in the liberation of its intracellular domain which translocates to the cell nucleus where it functions as a transcription factor. The implementation of this principle entails the co-introduction of two genes to the effector T cells. The first one is expressed constitutively and encodes such a chimeric cleavable receptor equipped with a recognition moiety directed at the first antigen. Engagement with this antigen on the surface of a target cell will turn on the expression of the second gene encoding a conventional CAR which is directed at the second antigen. The target cell will be killed only if it co-expresses this second antigen as well.

Inhibitory CARs. Off-tumor reactivity occurs when the target antigen of CAR-redirected killer cells is shared with normal tissue. If this normal tissue expresses another surface antigen not present on the tumor, then co-expressing in the gene-modified cells an additional CAR targeting this non-shared antigen, which harbors an inhibitory signaling moiety, can prevent T-cell activation by the normal tissue.

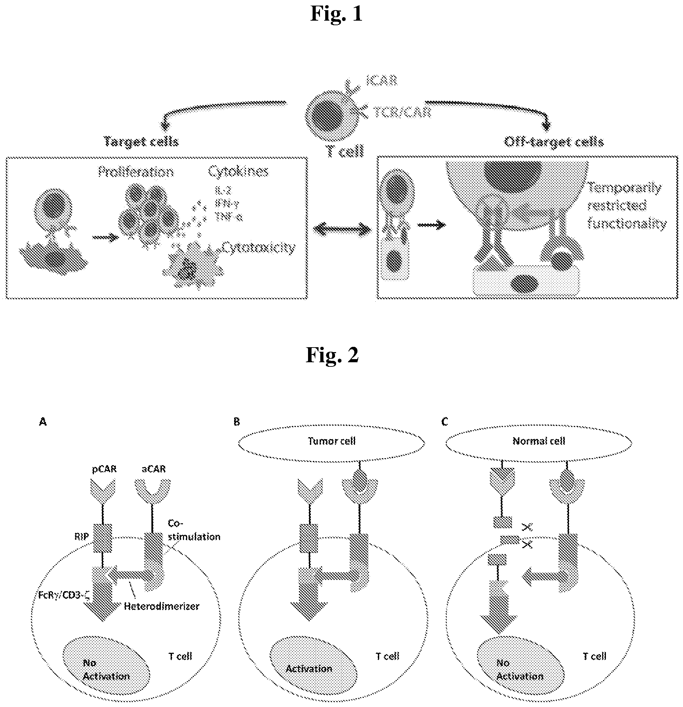

Instead of an activating domain (such as FcRy or CD3-ζ), an iCAR possesses a signaling domain derived from an inhibitory receptor which can antagonize T cell activation, such as CTLA-4, PD-1 or an NK inhibitory receptor. If the normal tissue which shares the candidate aCAR antigen with the tumor expresses another surface antigen not shared with the tumor, an iCAR expressed by the same T cell which targets this non-shared antigen can protect the normal tissue (Fig. 1).

Unlike T cells, each of which expresses a unique two-chain TCR encoded by somatically rearranged gene segments, NK cells do not express antigen-specific receptors. Instead, NK cells express an array of germline-encoded activating and inhibitory receptors which respectively recognize multiple activating and inhibitory ligands at the cell surface of infected and healthy cells. The protective capacity of an iCAR based on NK inhibitory receptors such as KIR3DL1 has been described (US 9,745,368). KIR3DL1 and other NK inhibitory receptors function by dismantling the immunological synapse in a rapid and comprehensive manner. There is compelling evidence that a single NK cell can spare a resistant cell expressing both inhibitory and activating ligands yet kill a susceptible cell it simultaneously engages, which expresses only the activating ligands (Abeyweera et al., 2011; Eriksson et al., 1999; Treanor et al., 2006; Vyas et al., 2001). This exquisite ability is governed by the different spatial organization of signal transduction molecules formed at each of the respective immune synapses which consequently affects the exocytosis of cytolytic granules (see (Huse et al., 2013) for review). More recently, Fedorov et al. (Fedorov et al., 2013a; WO 2015/142314) successfully employed for this purpose the intracellular domains of PD-1 and CTLA-4 Unlike NK inhibitory receptors, the regulatory effects of these iCARs affected the entire cell. Yet, these effects were temporary, allowing full T-cell activation upon subsequent encounter with target cells expressing only the aCAR antigen.

Tissue distribution of the antigens targeted by the iCAR and aCAR dictates the optimal mode of action of the iCAR required for conferring maximal safety without compromising clinical efficacy. For example, if the anatomical sites of the tumor and the normal tissue(s) to be protected do not intersect, transient inhibition (CTLA-4- or PD-1-like) will likely suffice. Yet, if these sites do overlap, only synapse-confined inhibition (i.e., an NK mode of action) will prevent constant paralysis of the therapeutic cells and allow their effective tumoricidal activity. The approach of using iCARs to reduce on-target off-tumor reactivity suffers from a dire lack of antigens downregulated in tumor cells but present on normal tissue.

Next generation sequencing (NGS) allows the determination of the DNA sequence of all protein-coding genes (~1% of the entire genome) in a given tumor biopsy and the comparison of the cancer 'exome' to that of a healthy tissue (usually from white blood cells) of the same patient. Exome sequencing can be completed within several days post-biopsy removal and at relatively low cost. In parallel, transcriptome analysis (RNA-seq) can provide complementary information on the genes that are actually expressed by the same cell sample.

It is becoming increasingly clear that the mutational landscape of each individual tumor is unique (Lawrence et al., 2013; Vogelstein et al., 2013). As a result of nonsynonymous mutations the tumor cell can potentially present a private set of neopeptides to the patient's immune system on one or more of his or her HLA products. Indeed, tremendous efforts are being put in recent years into identifying tumor-specific neoepitopes which can be recognized by the patient's own CDS or CD4 T cell repertoire and serve as targets for immunotherapy (for review see (Blankenstein et al., 2015; Van Buuren et al., 2014; Heemskerk et al., 2013; Overwijk et al., 2013; Schumacher and Schreiber, 2015)). However, cumulative findings suggest that neoantigen-based T cell immunotherapies are more likely to be effective in cancers displaying higher mutational load, such as melanoma and lung cancers, but may often fail to show benefit in most cancers with fewer mutations (Savage, 2014; Schumacher and Schreiber, 2015). Furthermore, considerable intratumoral heterogeneity (Burrell et al., 2013) entails the simultaneous co-targeting of several antigens so as to avoid emergence of mutation-loss variants, a task which becomes increasingly demanding in view of the scarcity of useful immunogenic neopeptides. Insert *6a

Leonardo Chicaybam et al., (2014, Cancer Research, vol 74, no. 19 Supplement, page 2797) and Leonardo Chicaybam et al., (2015, Cancer Research, Abstract 3156) describe a system comprising inhibitory and activating chimeric antigen receptors. The two documents disclose the creation of an inhibitory anti-CD20 CAR, that when used in combination with 19BBz (anti-CD19 CARs) would be able to discriminate between leukemic blasts and normal B cells.

Victor Fedorov et al., (2013, Science Translational Medicine, vol 5, no. 215) and WO2015/142314 (Sloan Kettering Inst Cancer) describe an inhibitory chimeric antigen receptor (iCAR) comprising an antigen recognition domain fused to signaling domains of immune inhibitory receptors CTLA-4 and PD1. The iCAR is shown to suppress antigen-specific T cell cytotoxicity, cytokine release, and proliferation and protect normal tissue from the off-target effects of T-cell therapy.

All in all, the urgent need to identify suitable targets for cancer immunotherapy via the adoptive transfer of genetically redirected killer cells is still largely unmet.

### SUMMARY OF INVENTION

The invention is defined by independent claim 1. Further embodiments of the invention are defined by the dependent claims.

In one aspect, the present invention provides a safe effector immune cell for use in treating cancer in a patient having a tumor characterized by LOH of a single allelic variant encoding a polymorphic cell surface epitope,
wherein the cell is transfected with a nucleic acid molecule comprising a nucleotide sequence encoding an inhibitory chimeric antigen receptor (iCAR) capable of preventing or attenuating undesired activation of an effector immune cell; and an intracellular domain comprising at least one signal transduction element that inhibits an effector immune cell, wherein the iCAR is directed to a single allelic variant encoding a polymorphic cell surface epitope absent from cells of the tumor due to loss of heterozygosity (LOH) but present at least on all cells of related mammalian normal tissue of the patient,
and wherein the polymorphic cell surface epitope is an HLA type I.

The description describes a method of preparing an inhibitory chimeric antigen receptor (iCAR) capable of preventing or attenuating undesired activation of an effector immune cell, according to the present invention as defined herein, the method comprising: (i) retrieving a list of human genomic variants of protein-encoding genes from at least one database of known variants; (ii) filtering the list of variants retrieved in (i) by: (a) selecting variants resulting in an amino acid sequence variation in the protein encoded by the respective gene as compared with its corresponding reference allele, (b) selecting variants of genes wherein the amino acid sequence variation is in an extracellular domain of the encoded protein, (c) selecting variants of genes that undergo loss of heterozygosity (LOH) at least in one tumor, and (d) selecting variants of genes that are expressed at least in a tissue of origin of the at least one tumor in which they undergo LOH according to (c), thereby obtaining a list of variants having an amino acid sequence variation in an extracellular domain in the protein encoded by the respective gene lost in the at least one tumor due to LOH and expressed at least in a tissue of origin of the at least one tumor; (iii) defining a sequence region comprising at least one single variant from the list obtained in (ii), sub-cloning and expressing the sequence region comprising the at least one single variant and a sequence region comprising the corresponding reference allele thereby obtaining the respective epitope peptides; (iv) selecting an iCAR binding domain, which specifically binds either to the epitope peptide encoded by the cloned sequence region, or to the epitope peptide encoded by the corresponding reference allele, obtained in (iii); and (vii) preparing iCARs as defined herein, each comprising an iCAR binding domain as defined in (iv).

The description describes a method for preparing a safe effector immune cell comprising: (i) transfecting a TCR-engineered effector immune cell directed to a tumor-associated antigen with a nucleic acid molecule comprising a nucleotide sequence encoding an iCAR as defined herein or transducing the cells with a vector defined herein, or (ii) transfecting a naive effector immune cell with a nucleic acid molecule comprising a nucleotide sequence encoding an iCAR as defined herein and a nucleic acid molecule comprising a nucleotide sequence encoding an aCAR as defined herein; or transducing an effector immune cell with a vector as defined herein.

The safe effector immune cell may be a redirected T cell expressing an exogenous T cell receptor (TCR) and an iCAR, wherein the exogenous TCR is directed to a non-polymorphic cell surface epitope of an antigen or a single allelic variant of a polymorphic cell surface epitope, wherein said epitope is a tumor-associated antigen or is shared at least by cells of related tumor and normal tissue, and the iCAR is as defined herein; or the safe effector immune cell is a redirected effector immune cell such as a natural killer cell or a T cell expressing an iCAR and an aCAR as defined herein.

The description describes a method of selecting a personalized biomarker for a subject having a tumor characterized by LOH, the method comprising (i) obtaining a tumor biopsy from the subject; (ii) obtaining a sample of normal tissue from the subject, e.g. peripheral blood mononuclear cells (PBMCs); and (iii) identifying a single allelic variant of a polymorphic cell surface epitope that is not expressed by cells of the tumor due to LOH, but that is expressed by the cells of the normal tissue, thereby identifying a personalized biomarker for the subject.

In a further aspect, the present invention provides a method for treating cancer in a patient having a tumor characterized by LOH, comprising administering to the patient an effector immune cell as defined herein, wherein the iCAR is directed to a single allelic variant encoding a polymorphic cell surface epitope absent from cells of the tumor due to loss of heterozygosity (LOH) but present at least on all cells of related mammalian normal tissue of the patient.

In still a further aspect, the present invention is directed to a safe effector immune cell as defined herein for use in treating a patient having a tumor characterized by LOH, wherein the iCAR is directed to a single allelic variant encoding a polymorphic cell surface epitope absent from cells of the tumor due to loss of heterozygosity (LOH) but present at least on all cells of related mammalian normal tissue of the patient.

In yet a further aspect, the present invention is directed to a method for treating cancer in a patient having a tumor characterized by LOH comprising: (i) identifying or receiving information identifying a single allelic variant of a polymorphic cell surface epitope that is not expressed by cells of the tumor due to LOH, but that is expressed by the cells of the normal tissue, (ii) identifying or receiving information identifying a non-polymorphic cell surface epitope of an antigen or a single allelic variant of a polymorphic cell surface epitope, wherein said epitope is a tumor-associated antigen or is shared by cells at least of related tumor and normal tissue in said cancer patient; (iii) selecting or receiving at least one nucleic acid molecule defining an iCAR as defined herein and at least one nucleic acid molecule comprising a nucleotide sequence encoding an aCAR as defined herein, or at least one vector as defined herein, wherein the iCAR comprises an extracellular domain that specifically binds to a cell surface epitope of (i) and the aCAR comprises an extracellular domain that specifically binds to a cell surface epitope of (ii); (iv) preparing or receiving at least one population of safe redirected effector immune cells by transfecting effector immune cells with the nucleic acid molecules of (iii) or transducing effector immune cells with the vectors of (iii); and (v) administering to said cancer patient at least one population of safe redirected immune effector cells of (iv).

In a similar aspect, the present invention provides at least one population of safe redirected immune effector cells for treating cancer in a patient having a tumor characterized by LOH, wherein the safe redirected immune cells are obtained by (i) identifying or receiving information identifying a single allelic variant of a polymorphic cell surface epitope that is not expressed by cells of the tumor due to LOH, but that is expressed by the cells of the normal tissue, (ii) identifying or receiving information identifying a non-polymorphic cell surface epitope of an antigen or a single allelic variant of a polymorphic cell surface epitope, wherein said epitope is a tumor-associated antigen or is shared by cells at least of related tumor and normal tissue in said cancer patient; (iii) selecting or receiving at least one nucleic acid molecule defining an iCAR as defined herein and at least one nucleic acid molecule comprising a nucleotide sequence encoding an aCAR as defined herein, or at least one vector as defined herein, wherein the iCAR comprises an extracellular domain that specifically binds to a cell surface epitope of (i) and the aCAR comprises an extracellular domain that specifically binds to a cell surface epitope of (ii); (iv) preparing or receiving at least one population of safe redirected effector immune cells by transfecting effector immune cells with the nucleic acid molecules of (iii) or transducing effector immune cells with the vectors of (iii).

The description describes a combination of two or more nucleic acid molecules, each one comprising a nucleotide sequence encoding a different member of a controlled effector immune cell activating system, said nucleic acid molecules being part of or forming a single continues nucleic acid molecule, or comprising two or more separate nucleic acid molecules, wherein the controlled effector immune activating system directs effector immune cells to kill tumor cells that have lost one or more chromosomes or fractions thereof due to Loss of Heterozygosity (LOH) and spares cells of related normal tissue, and wherein (a) the first member comprises an activating chimeric antigen receptor (aCAR) polypeptide comprising a first extracellular domain that specifically binds to a non-polymorphic cell surface epitope of an antigen or to a single allelic variant of a different polymorphic cell surface epitope and said non-polymorphic or polymorphic cell surface epitope is a tumor-associated antigen or is shared by cells of related abnormal and normal mammalian tissue; and (b) the second member comprises a regulatory polypeptide comprising a second extracellular domain that specifically binds to a single allelic variant of a polymorphic cell surface epitope not expressed by an abnormal mammalian tissue due to LOH but present on all cells of related mammalian normal tissue.

### BRIEF DESCRIPTION OF DRAWINGS

Fig. 1 shows the concept of iCARs (taken from (Fedorov et al., 2013a).
**Fig. 2** shows the aCAR/pCAR molecular design and mode of action. Binding of the pCAR to its antigen on normal cells, whether these express the aCAR antigen or not, is expected to result in rapid RIP and breaking of the polypeptide into 3 separate fragments.

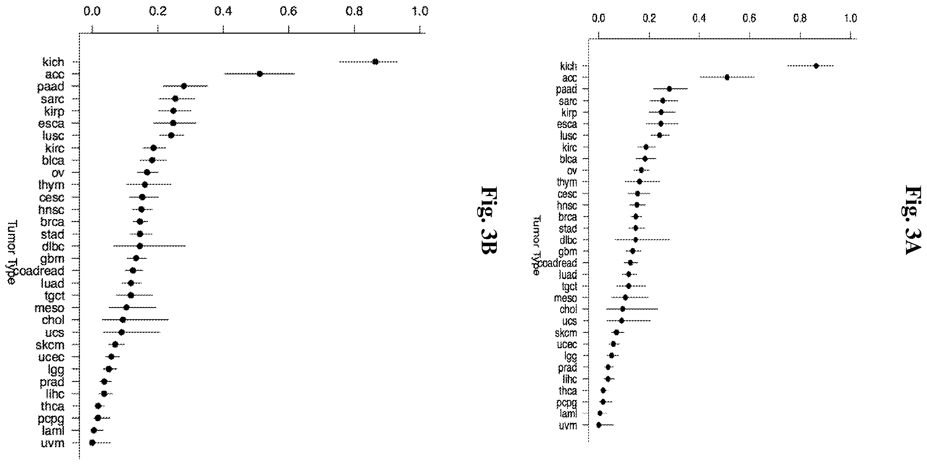

**Figs. 3A-C** show the percentage of tumor samples undergoing LOH in the chromosomal region coding for the HLA class I locus. **A**. HLA-G, **B**. HLA-A, C. ZNRD1, in tumor types from the TCGA database. Kidney Chromophobe [KICH], Adrenocortical carcinoma [ACC], Pancreatic adenocarcinoma [PAAD], Sarcoma [SARC], Kidney renal papillary cell carcinoma [KIRP], Esophageal carcinoma [ESCA], Lung squamous cell carcinoma [LUSC], Kidney renal clear cell carcinoma [KIRC], Bladder Urothelial Carcinoma [BLCA], Ovarian serous cystadenocarcinoma [OV], Thymoma [THYM], Cervical squamous cell carcinoma and endocervical adenocarcinoma [CESC], Head and Neck squamous cell carcinoma [HNSC], Breast invasive carcinoma [BRCA], Stomach adenocarcinoma [STAD], Lymphoid Neoplasm Diffuse Large B-cell Lymphoma [DLBC], Glioblastoma multiforme [GBM], Colon adenocarcinoma [COAD], Rectum adenocarcinoma [READ], Lung adenocarcinoma [LUAD], Testicular Germ Cell Tumors [TGCT], Mesothelioma [MESO], Cholangiocarcinoma [CHOL], Uterine Carcinosarcoma [UCS], Skin Cutaneous Melanoma [SKCM], Uterine Corpus Endometrial Carcinoma [UCEC], Brain Lower Grade Glioma [LGG], Prostate adenocarcinoma [PRAD], Liver hepatocellular carcinoma [LIHC], Thyroid carcinoma [THCA], Pheochromocytoma and Paraganglioma [PCPG], Acute Myeloid Leukemia [LAML], Uveal Melanoma [UVM]
**Figs. 4A-B** show the percentage of tumor samples undergoing LOH in the chromosomal region coding for the HLA class I locus. A. HLA-B, **B**. HLA-C, in the same tumor types of Fig. 1.

### DETAILED DESCRIPTION

Referring to the revolutionary concept of tumor suppressor genes (TSGs) that had been put forward in 1971 by A. G. Knudson (Knudson Jr., 1971), Devilee, Cleton-Jansen and Cornelisse stated in the opening paragraph of their essay titled 'Ever since Knudson' (Devilee et al., 2001): "Many publications have documented LOH on many different chromosomes in a wide variety of tumors, implicating the existence of multiple TSGs. Knudson's two-hit hypothesis predicts that these LOH events are the second step in the inactivation of both alleles of a TSG". In their seminal review on genetic instabilities in human cancers (Lengauer et al., 1998), Lengauer, Kinzler and Vogelstein wrote: "Karyotypic studies have shown that the majority of cancers have lost or gained chromosomes, and molecular studies indicate that karyotypic data actually underestimate the true extent of such changes. Losses of heterozygosity, that is, losses of a maternal or paternal allele in a tumor, are widespread and are often accompanied by a gain of the opposite allele. A tumor could lose the maternal chromosome 8, for example, while duplicating the paternal chromosome 8, leaving the cell with a normal chromosome 8 karyotype but an abnormal chromosome 8 'allelotype'. The 'average' cancer of the colon, breast, pancreas or prostate may lose 25% of its alleles and it is not unusual for a tumor to have lost over half of its alleles." These observations have since been reinforced and extended to almost all human cancers, including practically all carcinomas, in numerous reports (see (McGranahan et al., 2012) for review). It is now unambiguously established that nearly all individual tumors exhibit multiple losses of full chromosomes, entire chromosomal arms or sub-chromosomal regions of varying size. New algorithms are being rapidly developed (e.g. Sathirapongsasuti et al., 2011) for the determination of the LOH profile in any given cell sample based on the exome sequence data. While statistical bias may at present question the validity of some interpretations (Teo et al., 2012), such algorithms are likely to improve and replace most other methodologies for establishing LOH profiles which had been employed for this purpose in the pre-NGS era.

Early LOH events can be detected in premalignant cells of the same tissue, but not in surrounding normal cells (Barrett et al., 1999). LOH is irreversible and events can only accumulate, so that tumor heterogeneity reflects the accumulation of losses throughout tumor progression. While tumor subclones can develop which differ in later LOH events, the existence of a minimal LOH signature that is shared by premalignant cells, putative tumor stem cells and all tumor subclones in a given patient, is expected to be the rule. Branches stemming from this 'trunk' LOH pattern would still create a limited set of partially overlapping signatures which, together, cover all tumor cells in the same patient.

An inevitable outcome of gross LOH events is the concomitant loss of all other genes residing on the deleted chromosomal material, and these naturally include many genes encoding transmembrane proteins. Concerning their identity, a catalog of 3,702 different human cell surface proteins (the 'surfaceome') has been compiled (Da Cunha et al., 2009). The expression of ≈42% of surfaceome genes display broad tissue distribution while ≈85 genes are expressed by all tissues examined, which is the hallmark of housekeeping genes. These genes are candidates, the different polymorphic variants of which may serve as targets for the iCARs and a CARs of the present invention.

More recently, Bausch-Fluck et al. (Bausch-Fluck et al., 2015) applied their Chemoproteomic Cell Surface Capture technology to identify a combined set of 1492 cell surface glycoproteins in 41 human cell types. A large fraction of the surfaceome is expected to be expressed by any given tumor, each exhibiting a distinctive profile. Genes encoding cell surface proteins were found to be slightly enriched for single-nucleotide polymorphisms (SNPs) in their coding regions than all other genes (Da Cunha et al., 2009). Polymorphic in-frame insertions and deletions, which are rarer, further contribute to the number of variants and likely exert more robust structural effects on the polypeptide products than peptide sequence-altering (nonsynonymous) SNPs. Altogether, a typical genome contains 10,000 to 12,000 sites with nonsynonymous variants and 190-210 in-frame insertions/deletions (Abecasis et al., 2010; Auton et al., 2015). These variants are not evenly distributed throughout the genome as highly polymorphic genes such as the HLA locus (http://www.ebi.ac.uk/imgt/hla/stats.html) or certain G-protein-coupled receptor (GPCR) genes (Lee et al., 2003; Rana et al., 2001) create distinct variant 'hotspots'. Another layer of LOH-related hotspots stems from the frequent loss of certain chromosomes, or chromosome arms in different cancers (e.g., 3p and 17p in small-cell lung carcinoma (Lindblad-Toh et al., 2000), 17p and 18q in colorectal cancer (Vogelstein et al., 1989), 17q and 19 in breast cancer (Li et al., 2014; Wang et al., 2004) 9p in melanoma (Stark and Hayward, 2007), 10q in glioblastoma (Ohgaki et al., 2004) and more).

A significant fraction of allelic variations in surface proteins would affect the extracellular portion of the respective gene products, potentially creating distinct allele-restricted epitopes which, in principle, can be recognized and distinguished from other variants by highly-specific mAbs. It is well documented that mAbs can be isolated that discriminate between two variants of the same protein which differ in a single amino acid only (see, for example, an early example of mAbs that recognize point mutation products of the Ras oncogene with exquisite specificity (Carney et al., 1986)). Interestingly, it was shown that two mAbs specific to a single amino acid interchange in a protein epitope can use structurally distinct variable regions from their heavy and light chain V gene pools (Stark and Caton, 1991). Recently, Skora et al. (Skora et al., 2015) reported the isolation of peptide-specific scFvs which can distinguish between HLA-I-bound neopeptides derived from mutated KRAS and EGFR proteins and their wild type counterparts, differing in both cases in one amino acid.

All taken together, a unique antigenic signature of tumor cells emerges, that can allow their unequivocal discrimination from all other cells in the entire body of the individual patient. It comprises all transmembrane proteins encoded by allelic variants that are absent from the tumor cell surface owing to LOH but are present on normal cells of the cancer tissue of origin or other tissues expressing these genes. Naturally, each gene affected by LOH will be characterized by a distinct pattern of tissue distribution except for true housekeeping genes. The majority of these genes are not expected to be directly involved in tumorigenesis or maintenance of the transformed phenotype and, in this sense, their loss is of a 'passenger' nature.

The rationale presented above argues that a unique molecular portrayal is inevitably shaped by LOH for almost all tumors, which is marked by the absence of numerous polymorphic surface structures that are present on normal cells. Converting this postulated signature of the individual tumor to a targetable set of antigenic epitopes entails a practicable immunological strategy for translating the recognition of a particular 'absence' into an activating cue capable of triggering target cell killing. Importantly, the incorporation of a safety device to assure that on-target off-tumor reactivity is strictly avoided will be highly favorable in future clinical implementation of this strategy.

The present invention tackles this challenge through the co-expression in each therapeutic killer cell of a single pair of genes. One partner in this pair encodes an activating CAR, (aCAR) and the other encodes an inhibitory CAR (iCAR).

It should be emphasized that the present invention provides a new avenue enabling specific targeting of tumor cells while keeping the normal cells secure. The concept presented herein provides for the identification of new targets for iCARs (or pCARs), these targets defined as comprising single allelic variants of polymorphic cell surface epitopes, which are lost from tumor cells due to LOH of the chromosomal region they reside in, while remaining expressed on normal tissue. Because of the polymorphic variation, it is possible to distinguish the two alleles and target only the allele missing in the tumor cells. Further, the target antigen may not necessarily itself be a tumor suppressor gene, or a gene predicted to be involved with cancer, since it is chosen for being in a region lost by LOH and could therefore simply be linked to such genes. This is conceptually different from the methods employed or suggested to date in cancer therapy, which target tumor associated antigens or antigens downregulated at tumors regardless of polymorphism.

The distinction is crucial because the LOH, being a genomic event, results in a total loss of a specific variant from the tumor with a very rare probability of gaining back the lost allele. If the LOH event occurs very early in the development of tumors, it ensures a uniform target signature in all tumor cells derived from the initial pre-malignant tissue including metastatic tumors. Additionally LOH occurs in almost all types of cancer and this concept can therefore be relied upon as a universal tool for developing markers relevant to all these cancer types. Since the LOH events are to some extent random, the present invention further provides for selection of personalized tumor markers for each individual cancer patient, based on the specific LOH events which took place in that patient. The tools relied upon to execute this concept, the aCARs and the iCARs, are well-known and can be easily prepared using methods well-known in the art as taught for example, in WO 2015/142314, and in US 9,745,368

According to one strategy, the two CARs in every given pair specifically recognize the product of a different allelic variant of the same target gene for which the patient is heterozygous. The basic principle is as follows: the aCAR targets an allelic variant of a selected cell surface protein that is expressed by the given tumor cells and is not affected by LOH while the pCAR or iCAR targets the product encoded by the allelic variant of the same gene that has been lost from these tumor cells due to LOH In other normal tissues of that individual patient that express the said gene, both alleles are present and are known to be equally functional, that is, expression is biallelic in all tissues (in contrast to other genes which may exhibit random monoallelic expression (Chess, 2012; Savova et al., 2016). In one scenario, the two CARs target two related epitopes residing at the same location on the protein product, which differ by one, or only few amino acids. In another scenario, the aCAR targets a non-polymorphic epitope on the same protein while the pCAR or iCAR is allele-specific. In this case the density of the aCAR epitope on normal cells would generally be two-fold higher than that of the iCAR or pCAR one.

Another strategy utilizes as the pCAR or iCAR targets the protein products of housekeeping genes. Since, by definition, these genes are expressed on all cells in the body, they are safe targets for pCAR or iCARs. That is, if the pCAR or iCAR targets a membrane product of a housekeeping gene for which the given patient is heterozygous, all cells in the body, except the tumor cells which have lost this allele due to LOH, will be protected. This strategy allows for the uncoupling of the aCAR target gene product from the pCAR or iCAR one. In fact, the aCAR target can then be any non-polymorphic epitope expressed by the tumor. A variation of this strategy would be to utilize a known aCAR targeted to a non-polymorphic tumor-associated antigen, e.g. an aCAR in clinical use or under examination in clinical trials, in combination with an iCAR or pCAR directed against a membrane product of a gene for which the given patient is heterozygous and which is expressed in at least the tissue of origin of the tumor and preferably in additional vital normal tissues in which aCAR target antigen is expressed.

Care must be taken to ensure that the inhibitory signal transmitted by the iCAR is strictly and permanently dominant over the aCAR signal and that no cross-recognition between the iCAR and the aCAR occurs. Dominance of the iCAR guarantees that activation of the killer cell upon encounter with normal cells expressing both alleles would be prevented. This default brake would, however, not operate upon engagement with tumor cells: in the absence of its target antigen the iCAR would not deliver inhibitory signals, thus unleashing the anticipated aCAR-mediated cellular activation and subsequent tumor cell lysis.

The iCAR technology may be based on immune checkpoints. In this regard, the demonstration (Fedorov et al., 2013b; WO 2015/142314) that the regulatory elements of PD-1 and CTLA-4 possess a potent T cell inhibitory capacity when incorporated as iCAR signaling components is encouraging but the generality of these observations was recently questioned (Chicaybam and Bonamino, 2014, 2015). Furthermore, although the precise molecular pathways triggered by these checkpoint proteins are not fully understood, their engagement dampens T-cell activation through both proximal and distal mechanisms, rendering T cells unresponsive to concomitant activating stimuli (Nirschl and Drake, 2013). Hence, although the inactivation status secured by PD-1 and CTLA-4 iCARs is indeed temporary and reversible (Fedorov et al., 2013b), it would not allow T cell activation in tissues expressing both iCAR and aCAR targets. In contrast, the dominance of NK inhibitory receptors over activating receptors assures that healthy cells are spared from NK cell attack through a spatial, rather than temporal mechanism. (Long et al., 2013). There is compelling evidence that a single NK cell can spare a resistant cell expressing both inhibitory and activating ligands yet, kill a susceptible cell it simultaneously engages, which expresses only the activating ligands. This exquisite ability is governed by the different spatial organization of signal transduction molecules formed at each of the respective immune synapses which consequently affects the exocytosis of cytolytic granules (e.g., Abeyweera et al., 2011; Eriksson et al., 1999; Treanor et al., 2006; Vyas et al., 2001; US 9,745,368).

The strategy based on the control asserted by iCARs depends on the dominance of the iCAR activity over the aCAR activity as explained above. The description decribes the introduction of an entirely new type of iCAR, termed here a pCAR (for 'protective CAR, see Fig. 1), designed to operate in CAR T cells in a synapse-selective manner and guarantee full dominance over the co-expressed aCAR. It integrates two technological feats:
1. Uncoupling the activating moiety of the aCAR (FcRγ/CD3-ζ) from the recognition unit and the co-stimulatory element (e.g., CD28, 4-1BB) by genetically placing them on two different polypeptide products. Recoupling of these elements, which is mandatory for the aCAR function, will only take place by the addition of a heterodimerizing drug which can bridge the respective binding sites incorporated onto each of the polypeptides separately (Fig. 2B). The reconstruction of a fully functional CAR by bridging similarly split recognition and activating moieties by virtue of a heterodimerizing drug has recently been reported by Wu et al. (Wu et al., 2015). For this purpose these authors used the FK506 binding protein domain (FKBP, 104 amino acids) and the T2089L mutant of FKBP-rapamycin binding domain (FRB, 89 amino acids) that heterodimerize in the presence of the rapamycin analog AP21967 (**Scheme I**). This drug possess 1000-fold less immunosuppressive activity compared to rapamycin (Bayle et al., 2006; Graef et al., 1997; Liberies et al., 1997) and is commercially available (ARGENT^{™}, Regulated Heterodimerization Kit, ARIAD). 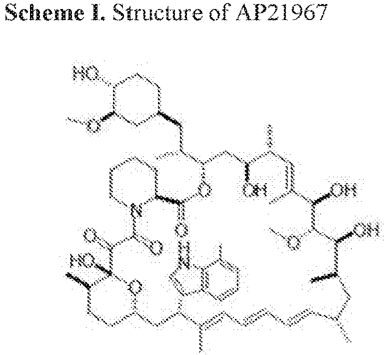
2. Engrafting the pCAR recognition unit and the missing activating domain, respectively, onto the two surfaces of the transmembrane domain of a RIP-controlled receptor which contains the two intramembrane cleavage sites (**Fig. 2A**). Binding of the pCAR to its antigen will trigger dual cleavage of the encoded polypeptide first by a member of the extracellular disintegrin and metalloproteinase (ADAM) family which removes the ectodomain and then by intracellular γ-secretase, which liberates the intracellular domain of the pCAR. This event is predicted to disrupt the ability of the truncated aCAR to gain access to a functional, membrane-anchored configuration of its missing activating element, thus acquiring an operative mode (**Fig. 2C**). This principle was recently exploited in the development of new genetic switches designed to limit CAR T cell activity to simultaneous recognition of two different antigens on the tumor cell, applying either the Notch receptor (Morsut et al., 2016; Roybal et al., 2016b) or Epithelial cell adhesion molecule (EpCAM, Pizem, Y., M.Sc. thesis under the supervision of the Inventor), two well-studied receptors functioning through RIP. In these studies, binding of the RIP-based CAR to one antigen releases a genetically-engineered intracellular domain which translocates to the cell nucleus where it turns on the expression of the second CAR. Unlike, the current invention utilizes this process solely for disarming any potential aCAR activity in the presence of the protective antigen.

The proposed mode of action described above is predicted to exert local effects so that only neighboring aCARs are affected and are no more able to bind their antigen productively and form an immunological synapse. As a result, even when multiple interactions of the aCAR with large numbers of non-tumor cells are likely to take place, they are only expected to be transient and nonfunctional so that the cells are fully capable of further interactions.

Dominance of the pCARs over their aCARs counterparts is inherent to this system as function of the aCARs utterly depends on presence of the pCARs. Relative shortage of pCARs in a given T cell would render the aCARs non-functional due to lack of an activating domain.

It is critical that both the recognition domain and the activating one are localized to the plasma membrane (Wu et al., 2015). Therefore, the 2^{nd} cleavage, which detaches the activating domain from the plasma membrane, would render this domain nonfunctional and prevent unwanted cellular activation.

The aCAR and pCAR are designed to function via mutually exclusive mechanisms. The ability of the pCAR to undergo cleavage does not depend on the strength of inhibitory signaling so no completion on signaling outcome will take place. As long as the pCARs are cleaved, the aCARs cannot function, regardless of relative avidity of their interactions with their respective antigens, a scenario which secures another crucial level of safety.

Any relevant technology may be used to engineer a recognition moiety that confers to the aCARs and pCAR or iCARs specific binding to their targets. For example, recognition moieties comprising this iCAR-aCAR Library may be derived from a master recognition moiety pool ideally selected from a combinatorial display library, so that:
- Collectively, the selected recognition moieties targets the cell-surface products of an array of genes which reside on each of the two arms of all 22 human autosomes. The shorter the distance between neighboring genes the fuller the coverage hence, the greater the universality of use.
- For each of the selected genes a set of allele-specific recognition moieties is isolated, each allowing rigorous discrimination between different allelic variants that are prevalent in the human population. The greater the number of targeted variants, the greater the number of therapeutic gene pairs that can be offered to patients.

A given allelic product can become a potential pCAR or iCAR target in one patient and a useful aCAR target in another patient harboring the same allele, depending on the particular LOH pattern in each case. Hence, as suitable recognition moiety genes are identified, each will be engrafted onto both a pCAR or an iCAR and an aCAR gene scaffold. It is therefore desirable that all recognition moieties directed at allelic variants of the same gene possess binding affinities of a similar range. Within such a given set of recognition moieties, all possible combinations of pCAR-aCAR or iCAR-aCAR pairs can be pre-assembled so as to assure the highest coverage of potential allelic compositions of that gene in the entire population.

In the more common scenario, the patient is heterozygous for the major allele and a minor one, the products of which differ in a single position along the encoded polypeptide as a result of a nonsynonymous SNP or, less frequently, an indel. In the less common scenario, a patient is heterozygous for two minor alleles which differ from the major one in two separate positions. Depending on the particular LOH event involving the said gene in individual patients, a given variant epitope can serve as an iCAR target in one patient and an aCAR target in another.

The identification of a variant-specific mAb (say, a mAb specific to the epitope encoded by the minor allele 'a') is well known in the art and is similar, in principle, to the identification of a mAb against any conventional antigenic determinant, and can usually best be accomplished via high throughput screening of a recombinant antibody scFv library, utilizing, for example, phage (Barbas et al., 2004), ribosome (Hanes et al., 1997) or yeast (Chao et al., 2006) display technologies. The antigen employed for library screening can either be a synthetic peptide spanning the position of variation between the two alleles (typically 15-20 amino acid in length or more), a recombinant full-length polypeptide which can either be commercially available or tailor-synthesized by one of the many companies operating in this field, or even entire cells expressing the said allelic variant at high level by virtue of gene transfection (e.g., electroporation of mRNA encoding the full-length cDNA cloned as template for in-vitro mRNA transcription in the pGEM4Z/A64 vector (Boczkowski et al., 2000)), following a subtraction step performed on the same cells not expressing this allele. These methods are well-known and described in e.g. Molecular Cloning: A Laboratory Manual (Fourth Edition) Green and Sambrook, Cold Spring Harbor Laboratory Press; Antibodies: A Laboratory Manual (Second Edition), Edited by Edward A. Greenfield, 2012 CSH laboratory press; Using Antibodies, A laboratory manual by Ed Harlow and David Lane, 1999 CSH laboratory press.

By definition, the corresponding epitope (at the same position) which is encoded by the major allele ('A'), creates a unique antigenic determinant that differs from that created by 'a' in the identity of a single amino acid (SNP) or length (indel). This determinant can, in principle, be recognized by a different set of mAbs identified by the same, or other, antibody display screening technology. The ability of distinct members in each of the two sets of identified mAbs to distinguish between the two epitopes, namely, an antibody from the first set binds the product of allele 'a' but not of 'A' and an Ab from the second set reciprocally binds 'A' but not 'a' can be determined using conventional binding assays such as ELISA or flow cytometry (Skora et al., 2015). Alternatively, once an 'a'-binding Ab is identified which does not bind 'A' and its protein sequence is determined, a computational method can potentially be used to predict the sequence of a 'complementary' antibody scFv which binds 'A' but not 'a'. For such a computational method see, for example (Sela-Culang et al., 2015a,b).

In the private example of presenting the HLA-class I locus genes HLA-A, HLA-B, and HLA-C as the target genes, there are numerous allele-specific monoclonal antibodies available, e.g. the antibodies listed in Example 3.

The sequences encoding the variable regions of these antibodies can easily be cloned from the relevant hybridoma and used for constructing genes encoding scFvs against specific HLA Class-I allelic epitope variants suitable for incorporation into a CAR construct using tools widely available as disclosed e.g. in Molecular Cloning: A Laboratory Manual (Fourth Edition) Green and Sambrook, Cold Spring Harbor Laboratory Press; Antibodies: A Laboratory Manual (Second Edition), Edited by Edward A. Greenfield, 2012 CSH laboratory press; Using Antibodies, A laboratory manual by Ed Harlow and David Lane, 1999 CSH laboratory press.

**The description describes** a database comprising DNA sequences of polymorphic variants lost in tumor cells due to LOH, and that encode cell-surface products, wherein the variation at the DNA sequence results in a variation at the amino acid sequence in an extracellular domain of the encoded protein. The information was retrieved from several databases open to the general public, such as TCGA, available on the public National Institute of Health TCGA data portal (https://gdc.cancer.gov/), which provides, *inter alia,* data that can be used to infer relative copy number of the gene in a variety of tumor types and the cbio portal for TCGA data at http://www.cbioportal.org (Cerami et al., 2012, Gao et al., 2013); the Exome Aggregation Consortium (ExAC) database (exac.broadinstitute.org, Lek et al., 2016), providing, *inter alia,* allele frequencies of SNP variants in various populations; the Genotype-Tissue Expression (GTEX) database v6p (dbGaP Accession phs000424.v6.pl) (https://gtexportal.org/home. Consortium GT. Human genomics, 2015) which includes tissue expression data for genes; and databases providing structural information of proteins, such as the Human Protein Atlas (Uhlen et al., 2015); the Cell Surface Protein Atlas (Bausch-Fluck et al., 2015), a mass-spectrometry based database of N-glycosylated cell-surface proteins, and the UniProt database (www.uniprot.org/downloads).

**The description describes** a method for genome-wide identification of genes that encode expressed cell-surface proteins that undergo LOH. The identified genes must meet the following criteria: 1)The gene encodes a transmembrane protein - therefore having a portion expressed on the cell surface to allow the iCAR binding; 2) The gene has at least two expressed alleles (in at least one ethnic population checked); 3) The allelic variation found for that gene causes an amino acid change relative to the reference sequence in an extracellular region of the protein; 4) The gene is located in a chromosomal region which undergoes LOH in cancer; 5) The gene is expressed in a tissue-of-origin of a tumor type in which the corresponding region was found to undergo LOH.

In principle genes as described above, suitable to encode targets for iCAR binding may be identified by any method known in the art, and not only by database mining. For example, the concept of LOH is not new and LOH information for specific genes, chromosomes, or genomic/chromosomal regions in specific tumors has already been published in the literature and candidate genes can therefore be derived from the available publications. Alternatively, such information can be found by whole genome hybridizations with chromosomal markers such as microsatellite probes (Medintz et al., 2000, Genmne Res. 2000 Aug; 10(8): 1211-1218) or by any other suitable method (Ramos and Amorim, 2015, J. Bras. Patol. Med. Lab. 51(3):198-196).

Similarly, information regarding allelic variants is publicly available in various databases, and can also be easily obtained for a personalized case by genomic sequencing of a suspected region. Also, information regarding protein structure and expression pattern is publicly available and easily accessible as described above.

Accordingly, as information regarding the various criteria for many genes and SNPs is publicly available and the techniques for retrieving it are generally known, the main novelty of the application is using LOH as a criterion for choosing a target for iCAR recognition, and the concept of personalizing treatment based on a specific allele lost in a specific patient.

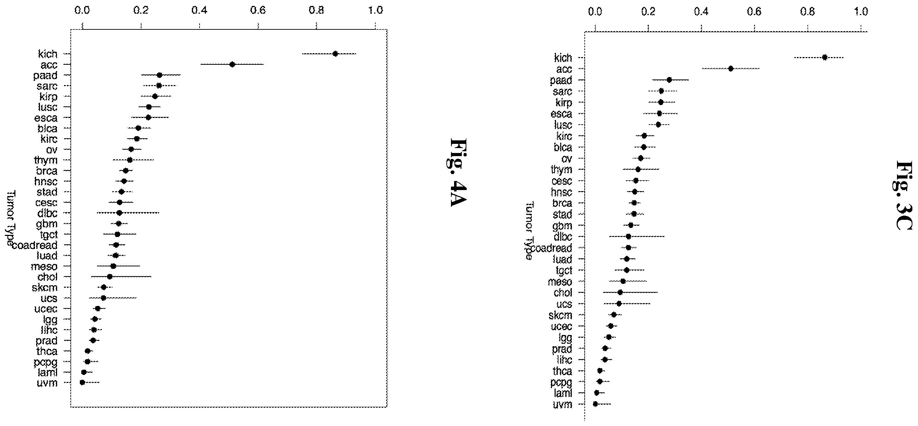

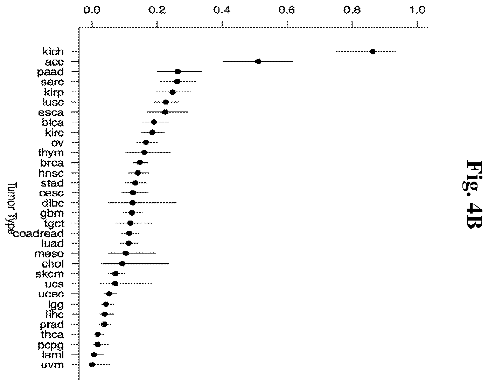

As a non-limiting example, it was found according to the present invention that HLA-A, HLA-B and HLA-C LOH, at varying frequencies, is a relatively frequent event in many tumor types (see Fig. 4), which would make these genes good candidates to be used as targets for iCAR recognition for the purpose of the present invention.

The recognition of the aCAR target on normal cells in any healthy essential tissue in the absence of the pCAR or iCAR target would be detrimental and is strictly forbidden. In this respect, the concept of pCAR-aCAR or iCAR-aCAR pairs, as proposed here, constitutes a fail-safe activation switch, as: i) cells not expressing the selected gene (in case the aCAR and the pCAR or iCAR target different products of the same gene) will not be targeted due to absence of the aCAR target antigen; ii) normal cells expressing this same gene will co-express both alleles and will not be targeted owing to the dominance of the pCAR or iCAR; iii) in case the pCAR or iCAR targets the product of a polymorphic housekeeping gene, all cells in the body will be protected. iv) only tumor cells which express the aCAR target but not the pCAR or iCAR one will be attacked.

As emphasized above, permanent dominance of the inhibitory signal over the activating one is absolutely mandatory. It is therefore crucial to ensure that no aCAR gene is expressed in a given killer cell, at any time, in the absence of its iCAR safeguard. This may be implemented through the tandem assembly of these iCAR-aCAR gene pairs as single-chain products or via a suitable bi-cistronic modality based, for example, on an internal ribosome entry site or on one of several viral self-cleaving 2A peptides. As suggested by the vast bulk of data reported on bi-cistronic expression, the iCAR gene will always be positioned upstream of its aCAR partner to guarantee favorable stoichiometry. Of course, this is not an issue when using a pCAR-aCAR gene pair.

Another attractive option for assuring iCAR dominance is detaching the aCAR recognition moiety from its activating/costimulatory portion so that both entities can only be assembled into one functional receptor in the presence of a heterodimerizing small molecule. The ability to tightly control the operative state of such split receptors by precise timing, dosage and location was recently demonstrated in the context of antitumor CARs (Wu et al., 2015).

In addition, the expected dominance is also likely to be intrinsic to the particular composition of the iCAR signaling elements incorporated into the intracellular portion in the selected iCAR design that should 'compete' with the signaling strength of the chosen aCAR platform. This capacity will also be influenced by the relative affinities of the two recognition moieties for their respective target epitopes (which was dealt with above) and the overall avidities of their interactions. Concerning the latter, the proposed strategy secures both a favorable iCAR/aCAR stoichiometry and a balanced distribution of their respective target epitopes on normal cells. Again, this is not an issue when using a pCAR-aCAR gene pair.

To further assure safety, other conventional means currently implemented in the field of CAR and TCR immunotherapy can be employed, such as the use of suicide genes or the use of mRNA electroporation for transient expression.

While LOH often leaves the cells with only one allele of a given gene, it is frequently accompanied by duplication of the remaining chromosome, or chromosome part, resulting in 'copy number neutral'-LOH (Lo et al., 2008; O'Keefe et al., 2010; Sathirapongsasuti et al., 2011). Under these circumstances, the emergence of epitope-loss variants requires two independent events and is thus less likely. Expressing several pCAR-aCAR or iCAR-aCAR pairs in different fractions of the gene-modified cells will prevent the appearance of mutational escapees even in 'copy number loss' LOH cases, in which only a single copy of the target allele has been retained. Yet, as single-copy genes may become essential, their functional loss would be far less likely.

**The description describes** a nucleic acid molecule comprising a nucleotide sequence encoding an inhibitory chimeric antigen receptor (iCAR) capable of preventing or attenuating undesired activation of an effector immune cell, wherein the iCAR comprises an extracellular domain that specifically binds to a single allelic variant of a polymorphic cell surface epitope absent from mammalian tumor cells due to loss of heterozygosity (LOH) but present at least on all cells of related mammalian normal tissue; and an intracellular domain comprising at least one signal transduction element that inhibits an effector immune cell.

In certain embodiments, the polymorphic cell surface epitope is part of an antigen encoded by a tumor suppressor gene or a gene genetically linked to a tumor suppressor gene, since such genes are likely to be lost due to LOH in tumors. Additionally, the polymorphic cell surface epitope may be part of an antigen encoded by a gene normally residing on a chromosome or chromosomal arm that often undergo LOH in cancer cells such as, but not limited to, chromosomal arms 3p, 6p, 9p, 10q, 17p, 17q, or 18q, or chromosome 19. These epitopes can readily be identified in the relevant databases as described herein.

In certain embodiments, the polymorphic cell surface epitope is of a housekeeping gene product, such as the unclassified AP2S1, CD81, GPAA1, LGALS9, MGAT2, MGAT4B, VAMP3; the cell adhesion proteins CTNNA1 NM_001903, CTNNB1, CTNNBIP1 NM_020248, CTNNBL1 NM_030877, CTNND1 NM_001085458 delta catenin; the channels and transporters ABCB10 NM_012089, ABCB7 NM_004299, ABCD3 NM_002857, ABCE1 NM_002939, ABCF1 NM_001090, ABCF2 NM_005692, ABCF3 NM_018358, CALM1[1][7] Calmodulin grasps calcium ions, MFSD1 1 NM 024311 similar to MSFD10 aka TETRAN or tetracycline transporter-like protein[1], MFSD12 NM_174983, MFSD3 NM_138431, MFSD5 NM_032889, SLC15A4 NM145648, SLC20A1 NM_005415, SLC25A11[1] mitochondrial oxoglutarate/malate carrier, SLC25A26 NM_173471, SLC25A28 NM_031212, SLC25A3 NM 002635, SLC25A32 NM_ 030780, SLC25A38 NM 017875, SLC25A39 NM 016016, SLC25A44 NM 014655, SLC25A46 NM 138773, SLC25A5 NM_001152, SLC27A4 NM 005094, SLC30A1 NM 021194, SLC30A5 NM 022902, SLC30A9 NM 006345, SLC35A2 NM 005660, SLC35A4 NM 080670, SLC35B1 NM 005827, SLC35B2 NM 178148, SLC35C2 NM 015945, SLC35E1 NM 024881, SLC35E3 NM_018656, SLC35F5 NM 025181, SLC38A2 NM_018976, SLC39A1 NM_014437, SLC39A3 NM 144564, SLC39A7 NM 006979, SLC41A3 NM_017836, SLC46A3 NM_181785, SLC48A1 NM_017842, the receptors ACVR1 NM_001105 similar to ACVRL1 TGF Beta receptor family Rendu-Osler-Weber syndrome, ACVR1B NM_004302,CD23[1] FCER2 low affinity IgE receptor (lectin); and the HLA/immunoglobulin/cell recognition group BAT1 aka DDX39B which is involved in RNA splicing, BSG Basigin Immunoglobulin Superfamily, extracelluar metalloproteinase, MIF macrophage migration inhibitory factor, TAPBP [Wikipedia],

In certain embodiments, the housekeeping gene is an HLA type I, a G-protein-coupled receptor (GPCR), an ion channel or a receptor tyrosine kinase, preferably an HLA-A, HLA-B or HLA-C.

Any relevant technology may be used to engineer a recognition moiety that confers to the aCARs and pCAR or iCARs specific binding to their targets. In certain embodiments, the extracellular domain comprises (i) an antibody, derivative or fragment thereof, such as a humanized antibody; a human antibody; a functional fragment of an antibody; a single-domain antibody, such as a Nanobody; a recombinant antibody; and a single chain variable fragment (ScFv); (ii) an antibody mimetic, such as an affibody molecule; an affilin; an affimer; an affitin; an alphabody; an anticalin; an avimer; a DARPin; a fynomer; a Kunitz domain peptide; and a monobody; or (iii) an aptamer. Preferably, the extracellular domain comprises an ScFv.

In certain embodiments, the mammalian tissue is human tissue and in other embodiments the related mammalian normal tissue is normal tissue from which the tumor developed.

In certain embodiments, the effector immune cell is a T cell, a natural killer cell or a cytokine-induced killer cell.

In certain embodiments, the at least one signal transduction element capable of inhibiting an effector immune cell is homologous to a signal transduction element of an immune checkpoint protein, such as an immune checkpoint protein selected from the group consisting of PD1; CTLA4; BTLA; 2B4; CD160; CEACAM, such as CEACAM1; KIRs, such as KIR2DL1, KIR2DL2, KIR2DL3, KIR2DL4, KIR2DL5A, KIR2DL5B, KIR3DL1, KIR3DL2). KIR3DL3, LIR1, LIR2, LIR3, LIR5, LIR8 and CD94-NKG2A; LAG3; TIM3; V-domain Ig suppressor of T cell activation (VISTA); STimulator of INterferon Genes (STING); immunoreceptor tyrosine-based inhibitory motif (ITIM)-containing proteins, T cell immunoglobulin and ITIM domain (TIGIT), and adenosine receptor (e.g. A2aR).

In certain embodiments, immune checkpoint protein is a natural killer cell inhibitory receptor, e.g. KIRs, such as KIR2DL1, KIR2DL2, KIR2DL3, KIR2DL4, KIR2DL5A, KIR2DL5B, KIR3DL1, KIR3DL2, KIR3DL3; or a Leukocyte Ig-like receptor, such as LIR1, LIR2, LIR3, LIR5, LIR8; and CD94-NKG2A, a C-type lectin receptor which forms heterodimers with CD94 and contains 2 ITIMs.

The methods for preparing and using killer cell receptors in iCARs has been described in US 9,745,368 .

In certain embodiments, the extracellular domain of any one of the above embodiments is fused through a flexible hinge and transmembrane canonic motif to said intracellular domain.

In certain embodiments, the iCAR is directed against or specifically binds to a single allelic variant of an antigen not including the ephrin receptors (e.g. EPHA 7) and claudins.

In certain embodiments, the iCAR is directed against or specifically binds to an epitope encoded by a single allelic variant of an HLA-A gene, HLA-B gene or HLA-C gene.

**The description describes** a vector comprising a nucleic acid molecule of the invention as defined in any one of the above embodiments, and at least one control element, such as a promoter, operably linked to the nucleic acid molecule.

In certain embodiments, the vector further comprises a nucleic acid molecule comprising a nucleotide sequence encoding an aCAR comprising an extracellular domain specifically binding a non-polymorphic cell surface epitope of an antigen or a single allelic variant of a polymorphic cell surface epitope, wherein said epitope is a tumor-associated antigen or is shared at least by cells of related tumor and normal tissue, and an intracellular domain comprising at least one signal transduction element that activates and/or co-stimulates an effector immune cell.

In certain embodiments, the extracellular domain of the aCAR encoded by the nucleic acid comprised in the vector specifically binds to a non-polymorphic cell surface epitope of an antigen and the extracellular domain of the iCAR specifically binds a single allelic variant of a polymorphic cell surface epitope of a different antigen than that to which the extracellular domain of said aCAR binds.

In certain embodiments, the extracellular domain of the iCAR encoded by the nucleic acid comprised in the vector, is directed against or specifically binds to a single allelic variant of an HLA-A gene, HLA-B gene or HLA-C gene.

In certain embodiments, the extracellular domain of the aCAR encoded by the nucleic acid comprised in the vector, is directed against or specifically binds to, a non-polymorphic cell surface epitope selected from the antigens listed in **Table 1,** such as CD19.

In certain embodiments, the extracellular domain of the iCAR encoded by the nucleic acid comprised in the vector, is directed against or specifically binds to a single allelic variant of an HLA-A gene, HLA-B gene or HLA-C gene; and the extracellular domain of the aCAR encoded by the nucleic acid comprised in the vector, is directed against or specifically binds to, a non-polymorphic cell surface epitope selected from the antigens listed in **Table 1,** such as CD19.

In certain embodiments, the at least one signal transduction element of the aCAR that activates or co-stimulates an effector immune cell is homologous to an immunoreceptor tyrosine-based activation motif (ITAM) of for example CD3ζ or FcRγ chains; a transmembrane domain of an activating killer cell immunoglobulin-like receptor (KIR) comprising a positively charged amino acid residue, or a positively charged side chain or an activating KIR transmembrane domain of e.g. KIR2DS and KIR3DS, or an adaptor molecule such as DAP12; or a co-stimulatory signal transduction element of for example CD27, CD28, ICOS, CD137 (4-1BB) or CD134 (OX40).

In certain embodiments, the nucleotide sequence of the vector comprises an internal ribosome entry site (IRES) between the nucleotide sequence encoding for the aCAR and the nucleotide sequence encoding for the iCAR. In general, the nucleotide sequence encoding for the aCAR and the nucleotide sequence encoding for the iCAR can be in any sequential order, but in particular embodiments, the nucleotide sequence encoding for the aCAR is downstream of the nucleotide sequence encoding for the iCAR.

In certain embodiments, the nucleotide sequence of the vector comprises a viral self-cleaving 2A peptide between the nucleotide sequence encoding for the aCAR and the nucleotide sequence encoding for the iCAR. In particular the viral self-cleaving 2A peptide may be selected from the group consisting of T2A from Thosea asigna virus (TaV), F2A from Foot-and-mouth disease virus (FMDV), E2A from Equine rhinitis A virus (ERAV) and P2A from Porcine teschovirus-1 (PTV1).

In certain embodiments, the vector comprises a nucleotide sequence encoding the constitutive aCAR linked via a flexible linker to said iCAR.

The immune cells may be transfected with the appropriate nucleic acid molecule described herein by e.g. RNA transfection or by incorporation in a plasmid fit for replication and/or transcription in a eukaryotic cell or a viral vector. In certain embodiments, the vector is selected from a retroviral or lentiviral vector.

Combinations of retroviral vector and an appropriate packaging line can also be used, where the capsid proteins will be functional for infecting human cells. Several amphotropic virus-producing cell lines are known, including PA12 (Miller, et al. (1985) Mol. Cell. BioI. 5:431-437); PA317 (Miller, et al. (1986) Mol. Cell. Bioi. 6:2895-2902); and CRIP (Danes, et ai. (1988) Proc. Nati. Acad. Sci. USA 85:6460-6464). Alternatively, non-amphotropic particles can be used, such as, particles pseudotyped with VSVG, RD 114, or GAL V envelope. Cells can further be transduced by direct, co-culture with producer cells, e.g., by the method of Bregni, et ai. (1992) Blood 80: 1418-1422, or culturing with viral supernatant alone or concentrated vector stocks, e.g., by the method of Xu, et ai. (1994) Exp. Hemat. 22:223-230; and Hughes, et ai. (1992) J Clin. Invest. 89: 1817.

**The description describes** a method of preparing an inhibitory chimeric antigen receptor (iCAR) capable of preventing or attenuating undesired activation of an effector immune cell, according to the present invention as defined above, the method comprising: (i) retrieving a list of human genomic variants of protein-encoding genes from at least one database of known variants; (ii) filtering the list of variants retrieved in (i) by: (a) selecting variants resulting in an amino acid sequence variation in the protein encoded by the respective gene as compared with its corresponding reference allele, (b) selecting variants of genes wherein the amino acid sequence variation is in an extracellular domain of the encoded protein, (c) selecting variants of genes that undergo loss of heterozygosity (LOH) at least in one tumor, and (d) selecting variants of genes that are expressed at least in a tissue of origin of the at least one tumor in which they undergo LOH according to (c), thereby obtaining a list of variants having an amino acid sequence variation in an extracellular domain in the protein encoded by the respective gene lost in the at least one tumor due to LOH and expressed at least in a tissue of origin of the at least one tumor; (iii) defining a sequence region comprising at least one single variant from the list obtained in (ii), sub-cloning and expressing the sequence region comprising the at least one single variant and a sequence region comprising the corresponding reference allele thereby obtaining the respective epitope peptides; (iv) selecting an iCAR binding domain, which specifically binds either to the epitope peptide encoded by the cloned sequence region, or to the epitope peptide encoded by the corresponding reference allele, obtained in (iii); and (vii) preparing iCARs as defined herein above, each comprising an iCAR binding domain as defined in (iv).

In certain embodiments, the candidate variants of genes that are selected undergo LOH in at least 2%, 5%, 10%, 20%, 30%, 40%, 50%, 60%, 70%, 80%, 90% or 100% in a certain tumor type.

In certain embodiments, the minor allele frequency for each variant selected equals or exceeds 1, 2, 3, 4 or 5% in at least one population.

**The description describes** a method for preparing a safe effector immune cell comprising: (i) transfecting a TCR-engmeered effector immune cell directed to a tumor-associated antigen with a nucleic acid molecule comprising a nucleotide sequence encoding an iCAR as defined herein above or transducing the cells with a vector of claim 9; or (ii) transfecting a naive effector immune cell with a nucleic acid molecule comprising a nucleotide sequence encoding an iCAR as defined herein above and a nucleic acid molecule comprising a nucleotide sequence encoding an aCAR as defined herein above, or transducing an effector immune cell with a vector as defined herein above.

**The description describes** a safe effector immune cell obtained by the method of the present invention as described above. The safe effector immune cell may be a redirected T cell expressing an exogenous T cell receptor (TCR) and an iCAR, wherein the exogenous TCR is directed to a non-polymorphic cell surface epitope of an antigen or a single allelic variant of a polymorphic cell surface epitope, wherein said epitope is a tumor-associated antigen or is shared at least by cells of related tumor and normal tissue, and the iCAR is as defined above, or the safe effector immune cell is a redirected effector immune cell such as a natural killer cell or a T cell expressing an iCAR and an aCAR. as defined above.

In certain embodiments, the safe effector immune cell, expresses on its surface an aCAR comprising an extracellular domain that specifically binds to a non-polymorphic cell surface epitope of an antigen and an iCAR comprising an extracellular domain that specifically binds a single allelic variant of a polymorphic cell surface epitope of a different antigen to which the extracellular domain of said aCAR binds. In certain embodiments, the extracellular domain of the iCAR specifically binds a single allelic variant of a different polymorphic cell surface epitope are of the same antigen to which the extracellular domain of said aCAR binds; or the extracellular domain of the iCAR specifically binds a different single allelic variant of the same polymorphic cell surface epitope area to which the extracellular domain of said aCAR binds.

In certain embodiments, the extracellular domain of the aCAR expressed on the cell surface specifically binds to a non-polymorphic cell surface epitope selected from the antigens listed in **Table 1**, such as CD19.

In certain embodiments, the extracellular domain of the iCAR expressed on the cell surface is directed against or specifically binds to a single allelic variant of an HLA-A gene, HLA-B gene or HLA-C gene.

In certain embodiments, the extracellular domain of the iCAR expressed on the cell surface is directed against or specifically binds to a single allelic variant of an HLA-A gene, HLA-B gene or HLA-C gene; and the extracellular domain of the aCAR expressed on the cell surface is directed against or specifically binds to, a non-polymorphic cell surface epitope selected from the antigens listed in **Table 1**, such as CD19.

In certain embodiments, the aCAR and the iCAR are present on the cell surface as separate proteins.

In certain embodiments, the expression level on the cell surface of the nucleotide sequence encoding the iCAR is greater than or equal to the expression level of the nucleotide sequence encoding the aCAR.

**The description describes** a method of selecting a personalized biomarker for a subject having a tumor characterized by LOH, the method comprising (i) obtaining a tumor biopsy from the subject; (ii) obtaining a sample of normal tissue from the subject, e.g. PBMCs; and (iii) identifying a single allelic variant of a polymorphic cell surface epitope that is not expressed by cells of the tumor due to LOH, but that is expressed by the cells of the normal tissue, thereby identifying a personalized biomarker for the subject.

In certain embodiments, the biomarker is used to customize a treatment of the subject, so the method further comprises the steps of treating cancer in a patient having a tumor characterized by LOH, comprising administering to the patient an effector immune cell as defined above, wherein the iCAR is directed to the single allelic variant identified in (iii).

**The description describes** a method for treating cancer in a patient having a tumor characterized by LOH, comprising administering to the patient an effector immune cell as defined above, wherein the iCAR is directed to a single allelic variant encoding a polymorphic cell surface epitope absent from cells of the tumor due to loss of heterozygosity (LOH) but present at least on all cells of related mammalian normal tissue of the patient.

**The description describes** a method of reducing tumor burden in a subject having a tumor characterized by LOH, comprising administering to the patient an effector immune cell as defined above, wherein the iCAR is directed to a single allelic variant encoding a polymorphic cell surface epitope absent from cells of the tumor due to loss of heterozygosity (LOH) but present at least on all cells of related mammalian normal tissue of the patient.

**The description describes** a method of increasing survival of a subject, having a tumor characterized by LOH, comprising administering to the patient an effector immune cell as defined above, wherein the iCAR is directed to a single allelic variant encoding a polymorphic cell surface epitope absent from cells of the tumor due to loss of heterozygosity (LOH) but present at least on all cells of related mammalian normal tissue of the patient.

**The description describes** a safe effector immune cell as defined above for use in treating, reducing tumor burden in, or increasing survival of, a patient having a tumor characterized by LOH, wherein the iCAR is directed to a single allelic variant encoding a polymorphic cell surface epitope absent from cells of the tumor due to loss of heterozygosity (LOH) but present at least on all cells of related mammalian normal tissue of the patient.

**The description describes** a method for treating cancer in a patient having a tumor characterized by LOH comprising: (i) identifying or receiving information identifying a single allelic variant of a polymorphic cell surface epitope that is not expressed by cells of the tumor due to LOH, but that is expressed by the cells of the normal tissue, (ii) identifying or receiving information identifying a non-polymorphic cell surface epitope of an antigen or a single allelic variant of a polymorphic cell surface epitope, wherein said epitope is a tumor-associated antigen or is shared by cells at least of related tumor and normal tissue in said cancer patient; (iii) selecting or receiving at least one nucleic acid molecule defining an iCAR as defined herein above and at least one nucleic acid molecule comprising a nucleotide sequence encoding an aCAR as defined herein above, or at least one vector as defined herein above, wherein the iCAR comprises an extracellular domain that specifically binds to a cell surface epitope of (i) and the aCAR comprises an extracellular domain that specifically binds to a cell surface epitope of (ii); (iv) preparing or receiving at least one population of safe redirected effector immune cells by transfecting effector immune cells with the nucleic acid molecules of (iii) or transducing effector immune cells with the vectors of (iii); and (v) administering to said cancer patient at least one population of safe redirected immune effector cells of (iv).

**The description describes** at least one population of safe redirected immune effector cells for treating cancer in a patient having a tumor characterized by LOH, wherein the safe redirected immune cells are obtained by (i) identifying or receiving information identifying a single allelic variant of a polymorphic cell surface epitope that is not expressed by cells of the tumor due to LOH, but that is expressed by the cells of the normal tissue, (ii) identifying or receiving information identifying a non-polymorphic cell surface epitope of an antigen or a single allelic variant of a polymorphic cell surface epitope, wherein said epitope is a tumor-associated antigen or is shared by cells at least of related tumor and normal tissue in said cancer patient; (iii) selecting or receiving at least one nucleic acid molecule defining an iCAR as defined herein above and at least one nucleic acid molecule comprising a nucleotide sequence encoding an aCAR as defined herein above, or at least one vector as defined herein above, wherein the iCAR comprises an extracellular domain that specifically binds to a cell surface epitope of (i) and the aCAR comprises an extracellular domain that specifically binds to a cell surface epitope of (ii); (iv) preparing or receiving at least one population of safe redirected effector immune cells by transfecting effector immune cells with the nucleic acid molecules of (iii) or transducing effector immune cells with the vectors of (iii).

In certain embodiments referring to any one of the above embodiments directed to treatment of cancer or safe immune effector cells for use in treatment of cancer, (i) the extracellular domain of the iCAR specifically binds a single allelic variant of a polymorphic cell surface epitope of an antigen, which is a different antigen than that to which the extracellular domain of the aCAR binds; (ii) the extracellular domain of said iCAR specifically binds a single allelic variant of a different polymorphic cell surface epitope of the same antigen to which the extracellular domain of said aCAR binds; or (iii) the extracellular domain of said iCAR specifically binds a different single allelic variant of the same polymorphic cell surface epitope to which the extracellular domain of said aCAR binds.

In certain embodiments, the treating results in reduced on-target, off-tumor reactivity, as compared with a treatment comprising administering to the cancer patient at least one population of immune effector cells expressing an aCAR of (iii) but lacking and iCAR of (iii).

In certain embodiments, the safe effector immune cells used for treating cancer as defined above express on their surface an aCAR comprising an extracellular domain that specifically binds to a tumor-associated antigen or a non-polymorphic cell surface epitope of an antigen and an iCAR comprising an extracellular domain that specifically binds a single allelic variant of a polymorphic cell surface epitope of an antigen expressed at least in a tissue of origin of the tumor or of a housekeeping protein, such as an HLA-A, HLA-B or HLA-C, which is a different antigen than that to which the extracellular domain of said aCAR binds.

In certain embodiments, more than one population of immune effector cells are administered, and the different populations express different pairs of aCARs and iCARs having specific binding to cell surface epitopes of different gene products.

In certain embodiments, the safe effector immune cells used in the method of treating cancer are selected from T cells, natural killer cells or cytokine-induced killer cells. In certain embodiments, the safe effector immune cell is autologous or universal (allogeneic) effector cells. In certain embodiments, the iCAR used in any one of the methods of treating cancer defined above is directed to all tissues of the patient on which the target-antigen of the aCAR is present, wherein the target antigen of the aCAR is a non-polymorphic cell surface epitope of an antigen or a single allelic variant of a polymorphic cell surface epitope is present, and said epitope is a tumor-associated antigen or is shared at least by cells of related tumor and normal tissue.

In certain embodiments, the cancer is selected from Acute Myeloid Leukemia [LAML], Adrenocortical carcinoma [ACC], Bladder Urothelial Carcinoma [BLCA], Brain Lower Grade Glioma [LGG], Breast invasive carcinoma [BRCA], Cervical squamous cell carcinoma and endocervical adenocarcinoma [CESC], Cholangiocarcinoma [CHOL], Colon adenocarcinoma [COAD], Esophageal carcinoma [ESCA], Glioblastoma multiforme [GBM], Head and Neck squamous cell carcinoma [HNSC], Kidney Chromophobe [KICH], Kidney renal clear cell carcinoma [KIRC], Kidney renal papillary cell carcinoma [KIRP], Liver hepatocellular carcinoma [LIHC], Lung adenocarcinoma [LUAD], Lung squamous cell carcinoma [LUSC], Lymphoid Neoplasm Diffuse Large B-cell Lymphoma [DLBC], Mesothelioma [MESO], Ovarian serous cystadenocarcinoma [OV], Pancreatic adenocarcinoma [PAAD], Pheochromocytoma and Paraganglioma [PCPG], Prostate adenocarcinoma [PRAD], Rectum adenocarcinoma [READ], Sarcoma [SARC], Skin Cutaneous Melanoma [SKCM], Stomach adenocarcinoma [STAD], Testicular Germ Cell Tumors [TGCT], Thymoma [THYM], Thyroid carcinoma [THCA], Uterine Carcinosarcoma [UCS], Uterine Corpus Endometrial Carcinoma [UCEC], Uveal Melanoma [UVM].

In certain embodiments, the iCAR used to treat the cancer, such as any one of the cancer types recited above, is directed against or specifically binds to a single allelic variant of an HLA-A gene, HLA-B gene or HLA-C gene.

In certain embodiments, the aCAR used to treat the cancer, such as any one of the cancer types recited above, is directed against or specifically binds to, a non-polymorphic cell surface epitope selected from the antigens listed in **Table 1**, such as CD19.

In certain embodiments, the iCAR used to treat the cancer, such as any one of the cancer types recited above, is directed against or specifically binds to a single allelic variant of an HLA-A gene, HLA-B gene or HLA-C gene; and the aCAR used to treat the cancer, such as any one of the cancer types recited above, is directed against or specifically binds to, a non-polymorphic cell surface epitope selected from the antigens listed in **Table 1,** such as CD19.

**The description describes** a combination of two or more nucleic acid molecules, each one comprising a nucleotide sequence encoding a different member of a controlled effector immune cell activating system, said nucleic acid molecules being part of or forming a single continues nucleic acid molecule, or comprising two or more separate nucleic acid molecules, wherein the controlled effector immune activating system directs effector immune cells to kill tumor cells that have lost one or more chromosomes or fractions thereof due to Loss of Heterozygosity (LOH) and spares cells of related normal tissue, and wherein (a) the first member comprises an activating chimeric antigen receptor (aCAR) polypeptide comprising a first extracellular domain that specifically binds to a non-polymorphic cell surface epitope of an antigen or to a single allelic variant of a different polymorphic cell surface epitope and said non-polymorphic or polymorphic cell surface epitope is a tumor-associated antigen or is shared by cells of related abnormal and normal mammalian tissue; and (b) the second member comprises a regulatory polypeptide comprising a second extracellular domain that specifically binds to a single allelic variant of a polymorphic cell surface epitope not expressed by an abnormal mammalian tissue due to LOH but present on all cells of related mammalian normal tissue.

In certain embodiments, the first member is selected from: (a) a constitutive aCAR further comprising an intracellular domain comprising at least one signal transduction element that activates and/or co-stimulates an effector immune cell; and (b) a conditional aCAR further comprising an intracellular domain comprising a first member of a binding site for a heterodimerizing small molecule and optionally at least one co-stimulatory signal transduction element, but lacking an activating signal transduction element; and the second member is: (c) an inhibiting chimeric antigen receptor (iCAR) further comprising an intracellular domain comprising at least one signal transduction element that inhibits an effector immune cell; or (d) a protective chimeric antigen receptor (pCAR) further comprising an extracellular regulatory region comprising a substrate for a sheddase; a transmembrane canonic motif comprising a substrate for an intramembrane-cleaving protease; and an intracellular domain, said intracellular domain comprising at least one signal transduction element that activates and/or co-stimulates an effector immune cell and a second member of a binding site for a heterodimerizing small molecule.

In certain embodiments (i) the extracellular domain of the iCAR or pCAR specifically binds a single allelic variant of a polymorphic cell surface epitope of an antigen, which is a different antigen than that to which the extracellular domain of the aCAR binds; (ii) the extracellular domain of said pCAR or iCAR specifically binds a single allelic variant of a different polymorphic cell surface epitope of the same antigen to which the extracellular domain of said aCAR binds; or (iii) the extracellular domain of said pCAR or iCAR specifically binds a different single allelic variant of the same polymorphic cell surface epitope to which the extracellular domain of said aCAR binds.

In certain embodiments, the substrate for a sheddase is a substrate for a disintegrin and metalloproteinase (ADAM) or a beta-secretase 1 (BACE1). In certain embodiments, the substrate forms part of the extracellular domain and comprises Lin 12/Notch repeats and an ADAM protease cleavage site.

It is generally accepted that there is no consistent sequence motif predicting ADAM cleavage, but Caescu et al. (Caescu et al., 2009) disclose in Table 2 a large number of ADAM10 and/or ADAM17 substrate sequences.
and which may serve as a substrate for ADAM in the pCAR of the present invention. In certain embodiments, the ADAM substrate sequences are those of amyloid precursor protein, BTC, CD23, Collagen, DII-1, Ebola glycoprotein, E-cadherin, EGF, Epiregulin, Fas Ligand, growth hormone receptor, HB-EGF, type II interleukin-1 receptor, IL-6 receptor, L-selectin, N-cadherin, Notch, p55 TNF receptor, p75 TNF receptor, Pmel17, Prion protein, receptor-type protein tyrosine phosphatase Z, TGF-α, TNF or TR (Caescu et al., 2009).

It may be advantageous to use an ADAM10 cleavage sequence in the pCAR of the present invention because ADAM 10 is constitutively present at comparably high levels on e.g. lymphocytes. In contrast to ADAM 10, the close relative TACE/ADAM17 is detected at only low levels on unstimulated cells. ADAM17 surface expression on T cell blasts is rapidly induced by stimulation (Ebsen et al., 2013).

Hemming et al. (Hemming et al., 2009) report, that no consistent sequence motif predicting BACE1 cleavage has been identified in substrates versus non-substrates, but discloses in Table 1 a large number of BACE1 substrates having BACl cleavage sequences,
and which may serve as a substrate for BACE1 in the pCAR of the present invention.

In certain embodiments, the substrate for an intramembrane-cleaving protease is a substrate for an SP2, a γ -secretase, a signal peptide peptidase (spp), a spp-like protease or a rhomboid protease.

Rawson et al. (Rawson, 2013) disclose that SP2 substrates have at least one type 2 membrane-spanning helix and include a helix-destabilizing motif, such as an Asp-Pro motif in a SP2 substrate. This paper discloses in Table 1 a number of SP2 substrates having SP2-cleavage sequences, and which may serve as a substrate for SP2 in the pCAR of the present invention.

Haapasalo and Kovacs (Haapasalo and Kovacs, 2011) teach that amyloid-β protein precursor (AβPP) is a substrate for presenilin (PS)-dependent γ-secretase (PS/γ-secretase), and that at least 90 additional proteins have been found to undergo similar proteolysis by this enzyme complex. γ-secretase substrates have some common features: most substrate proteins are type-I transmembrane proteins; the PS/γ-secretase-mediated γ-like cleavage (corresponding to the ε-cleavage in AβPP, which releases AICD) takes place at or near the boundary of the transmembrane and cytoplasmic domains. The ε-like cleavage site flanks a stretch of hydrophobic amino acid sequence rich in lysine and/or arginine residues. It appears that PS/γ-secretase cleavage is not dependent on a specific amino acid target sequence at or adjacent to the cleavage site, but rather perhaps on the conformational state of the transmembrane domain. Haapasalo and Kovacs disclose in Table 1 a list of γ-secretase substrates, the cleavage sequences and which may serve as a substrate for γ-secretases in the pCAR of the present invention.

Voss et al. (Voss et al., 2013) teach that so far no consensus cleavage site based on primary sequence elements within the substrate has been described for GxGD aspartyl proteases (spps). Transmembrane domains of membrane proteins preferentially adopt an α-helical confirmation in which their peptide bonds are hardly accessible to proteases. In order to make transmembrane domains susceptible for intramembrane proteolysis it was therefore postulated that their α-helical content needs to be reduced by helix destabilizing amino acids. Consistent with this hypothesis, various signal peptides have been shown to contain helix destabilizing amino acids within their h-region which critically influence their proteolytic processing by SPP. In addition, polar residues within the h-region of signal peptides may influence cleavage by SPP, as for instance serine and cysteine residues within the signal peptide of various HCV strains are critical for SPP cleavage. Whether these polar residues also simply affect the helical content of the signal peptides or the hydroxyl or sulfhydryl group in particular is required to trigger cleavage by SPP is not yet fully understood. Similarly, cleavage of the Bri2 transmembrane domain by SPPL2b is significantly increased when the α-helical content of the Bri2 transmembrane domainis reduced. Interestingly, only one amino acid residue out of four residues with a putative helix destabilizing potency significantly reduced the α-helical content of the Bri2 transmembrane domainin a phospholipid based environment. This suggests that destabilization of an α-helical transmembrane domain is not simply caused by certain amino acid residues but that rather context and position of these amino acids determine their helix destabilizing potential and thus the accessibility of transmembrane domains to intramembrane proteolysis by SPP/SPPLs. Voss et al. further disclose in Table 1 a list of spp and spp-like substrates, the cleavage sequences
and which may serve as a substrate for spp in the pCAR of the present invention.

Bergbold et al. (Bergbold and Lemberg, 2013) teach that for rhomboid proteases, two different models for substrate recognition have been suggested. In the first model, the conformational flexibility of the substrate peptide backbone combined with immersion of the membrane in the vicinity of the rhomboid active site is sufficient to provide specificity. For the well-characterized Drosophila substrate Spitz, a glycine- alanine motif has been shown to serve as a helix break that allows unfolding of the transmembrane domain into the rhomboid active site. The second model suggests that rhomboid proteases primarily recognize a specific sequence surrounding the cleavage site, and that transmembrane helix-destabilizing residues are a secondary feature required for some substrates only. The specific sequence has not yet been identified. Bergbold et al. disclose in Table 2 a list of rhomboid protease substrates, the cleavage sequences and which may serve as a substrate for rhomboid proteases in the pCAR of the present invention.

In view of the above, since in most cases no consensus motif has yet been established for the intramembrane-cleaving proteases, and since assays for identifying intramembrane-cleaving protease substrates are well known in the art as described in literature cited herein above, the pCAR may comprise an amino acid sequence identified as such and may further comprise transmembrane helix-destabilizing residues.

In certain embodiments, the substrate forms part of the transmembrane canonic motif and is homologous to/derived from a transmembrane domain of Notch, ErbB4, E-cadherin, N-cadherin, ephrin-B2, amyloid precursor protein or CD44.

In certain embodiments, the comprises a nucleotide sequence encoding an extracellular domain and an intracellular domain of said conditional aCAR as separate proteins, wherein each domain is independently fused to a transmembrane canonic motif and comprises a different member of a binding site for a heterodimerizing small molecule.

In certain embodiments, the each one of the first and second member of the binding site for a heterodimerizing small molecule is derived from a protein selected from: (i) Tacrolimus (FK506) binding protein (FKBP) and FKBP; (ii) FKBP and calcineurin catalytic subunit A (CnA); (iii) FKBP and cyclophilin; (iv) FKBP and FKBP-rapamycin associated protein (FRB); (v) gyrase B (GyrB) and GyrB; (vi) dihydrofolate reductase (DHFR) and DHFR; (vii) DmrB homodimerization domain (DmrB) and DmrB; (viii) a PYL protein (a.k.a. abscisic acid receptor and as RCAR) and ABI; and (ix) GAI *Arabidopsis thaliana* protein (a.k.a Gibberellic Acid Insensitive and DELLA protein GAI; GAI) and GID1 *Arabidopsis thaliana* protein (also known as Gibberellin receptor GID1; GID1).

### Definitions:

The term "nucleic acid molecule" as used herein refers to a DNA or RNA molecule.

The term "genomic variant" as used herein refers to a change of at least one nucleotide at the genomic level in a sequenced sample compared to the reference or consensus sequence at the same genomic position.

The term "corresponding reference allele" as used herein with reference to a variant means the reference or consensus sequence or nucleotide at the same genomic position as the variant.

The term "extracellular domain" as used herein with reference to a protein means a region of the protein which is outside of the cell membrane.

The term "loss of heterozygosity" or "LOH" as used herein means the loss of chromosomal materials such as a complete chromosome or a part thereof, in one copy of the two chromosomes in a somatic cell.

The term "sequence region" as used herein with reference to a variant or a reference allele means a sequence starting upstream and ending downstream from the position of the variant, which can be translated into an "epitope peptide" that can be recognized by an antibody.

The term "specific binding" as used herein in the context of an extracellular domain, such as an scFv, that specifically binds to a single allelic variant of a polymorphic cell surface epitope, refers to the relative binding of the scFv to one allelic variant and it's failure to bind to the corresponding different allelic variant of the same polymorphic cell surface epitope. Since this depends on the avidity (number of CAR copies on the T cell, number of antigen molecules on the surface of target cells (or cells to be protected) and the affinity of the specific CARs used, a functional definition would be that the specific scFv would provide a significant signal in an ELISA against the single allelic variant of a polymorphic cell surface epitope to which it is specific or cells transfected with a CAR displaying the scFv would be clearly labeled with the single allelic variant of a polymorphic cell surface epitope in a FACS assay, while the same assays using the corresponding different allelic variant of the same polymorphic cell surface epitope would not give any detectable signal.

The term "treating" as used herein refers to means of obtaining a desired physiological effect. The effect may be therapeutic in terms of partially or completely curing a disease and/or symptoms attributed to the disease. The term refers to inhibiting the disease, i.e. arresting its development; or ameliorating the disease, i.e. causing regression of the disease.

As used herein, the terms "subject" or "individual" or "animal" or "patient" or "mammal," refers to any subject, particularly a mammalian subject, for whom diagnosis, prognosis, or therapy is desired, for example, a human.

Pharmaceutical compositions for use in accordance with the present invention may be formulated in conventional manner using one or more physiologically acceptable carriers or excipients. The carrier(s) must be "acceptable" in the sense of being compatible with the other ingredients of the composition and not deleterious to the recipient thereof.

The following exemplification of carriers, modes of administration, dosage forms, etc., are listed as known possibilities from which the carriers, modes of administration, dosage forms, etc., may be selected for use with the present invention. Those of ordinary skill in the art will understand, however, that any given formulation and mode of administration selected should first be tested to determine that it achieves the desired results.

Methods of administration include, but are not limited to, parenteral, e.g., intravenous, intraperitoneal, intramuscular, subcutaneous, mucosal (e.g., oral, intranasal, buccal, vaginal, rectal, intraocular), intrathecal, topical and intradermal routes. Administration can be systemic or local. In certain embodiments, the pharmaceutical composition is adapted for oral administration.

The term "carrier" refers to a diluent, adjuvant, excipient, or vehicle with which the active agent is administered. The carriers in the pharmaceutical composition may comprise a binder, such as microcrystalline cellulose, polyvinylpyrrolidone (polyvidone or povidone), gum tragacanth, gelatin, starch, lactose or lactose monohydrate; a disintegrating agent, such as alginic acid, maize starch and the like; a lubricant or surfactant, such as magnesium stearate, or sodium lauryl sulphate; and a glidant, such as colloidal silicon dioxide.

For oral administration, the pharmaceutical preparation may be in liquid form, for example, solutions, syrups or suspensions, or may be presented as a drug product for reconstitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, or fractionated vegetable oils); and preservatives (e.g., methyl or propyl-p-hydroxybenzoates or sorbic acid). The pharmaceutical compositions may take the form of, for example, tablets or capsules prepared by conventional means with pharmaceutically acceptable excipients such as binding agents (e.g., pregelatinized maize starch, polyvinyl pyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well-known in the art.

Preparations for oral administration may be suitably formulated to give controlled release of the active compound.

For buccal administration, the compositions may take the form of tablets or lozenges formulated in conventional manner.

The compositions may be formulated for parenteral administration by injection, e.g., by bolus injection or continuous infusion. Formulations for injection may be presented in unit dosage form, e.g., in ampoules or in multidose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen free water, before use.

The compositions may also be formulated in rectal compositions such as suppositories or retention enemas, e.g., containing conventional suppository bases such as cocoa butter or other glycerides.

For administration by inhalation, the compositions for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin, for use in an inhaler or insufflator may be formulated containing a powder mix of the compound and a suitable powder base such as lactose or starch.

The term "peripheral blood mononuclear cell (PBMC)" as used herein refers to any blood cell having a round nucleus, such as a lymphocyte, a monocyte or a macrophage. Methods for isolating PBMCs from blood are readily apparent to those skilled in the art. An non-limiting example is the extraction of these cells from whole blood using ficoll, a hydrophilic polysaccharide that separates layers of blood, with monocytes and lymphocytes forming a buffy coat under a layer of plasma or by leukapheresis, the preparation of leukocyte concentrates with the return of red cells and leukocyte-poor plasma to the donor.

For purposes of clarity, and in no way limiting the scope of the teachings, unless otherwise indicated, all numbers expressing quantities, percentages or proportions, and other numerical values recited herein, should be interpreted as being preceded in all instances by the term "about." Accordingly, the numerical parameters recited in the present specification are approximations that may vary depending on the desired outcome. For example, each numerical parameter may be construed in light of the number of reported significant digits and by applying ordinary rounding techniques.

The term "about" as used herein means that values of 10% or less above or below the indicated values are also included.

### EXAMPLES

### Example 1. Genome-wide identification of polymorphic genes that encode expressed cell-surface proteins and undergo loss of heterozygosity (LOH)

In order to identify genes which can serve as iCAR target, the following requirements were employed:
1. The gene encodes a transmembrane protein - therefore having a portion expressed on the cell surface to allow the iCAR binding.
2. The gene has at least two expressed alleles (in at least one ethnic population checked)
3. The allelic variation found for that gene causes an amino acid change relative to the reference sequence in an extracellular region of the protein,
4. The gene is located in a chromosomal region which undergoes LOH in cancer.
5. The gene is expressed in a tissue-of-origin of a tumor type in which the corresponding region was found to undergo LOH.

### Allele identification:

The Exome Aggregation Consortium database (ExAC, at exac.broadinstitute.org) was used as an input to the analysis. The ExAC database is a compilation of exomes from various population-level sequencing studies totaling 60,706 exomes (Lek et al., 2016). ExAC contains information about each variant including the number of counts of the reference allele compared to the number of counts of the variant allele (allelic frequency - the number of counts of the variant allele out of the total number of chromosomes). The allelic frequency information is extended to subpopulations within the database as detailed in Table 2, the threshold was allelic frequency of 5% or more in at least one population.

**Table 2. Subpopulations within the ExAC database.**

| **Population ancestry** | **Population Abbreviation** | **Number of Individuals** |
|---|---|---|
| African | AFR | 5,203 |
| Latino | AMR | 5,789 |
| East Asian | EAS | 4,327 |
| Finnish | FIN | 3,307 |
| Non-Finnish European | NFE | 33,370 |
| South Asian | SAS | 8,256 |
| Other | OTH | 454 |

As per the requirements listed above, the following filters were applied to variants retrieved from the ExAC database:
1) the variant must affect the amino acid composition of the encoded protein ii) the variant must have a minor allele frequency equal to or greater than 0.05 (5%) in at least one population which appears in the ExAC database. The analysis was corrected for scenarios where the minor allele had an allele fraction greater than 0.5 (50%). If more than three alleles at a site were observed, then the most prevalent substitution was used.
2) A variant (in this case single nucleotide polymorphism (SNP)) was annotated as having an impact on the composition of the protein if the variation was classified into any of the following variant classes: 'missense_variant', 'inframe_deletion', 'start_lost','stop_gained', 'inframe_insertion', 'stop_retained variant', 'frameshift_variant', 'stop lost', 'coding_sequence_variant', 'protein_altering_variant'.

The analysis started with 9,362,319 SNP Variants, out of which 29,904 variants passed these above two filters. These variants fell in **10,322** genes having alleles with a minor allele frequency of equal to or greater than 5%, and which have an impact on the protein amino acid sequence. All alleles matching these two filters were included in the analysis.

### Identification of expressed genes:

The Genotype-Tissue Expression (GTEX) database v6p (dbGaP Accession phs000424.v6.p1) was used for the identification of genes that are expressed in various tissue types (https://gtexportal.org/home, Consortium GT. Human genomics, 2015). The GTEX database consists of RNA-sequencing of 8,555 human samples from diverse healthy tissue types.

The mean expression level of each gene in the tissue of origin corresponding to each tumor type for which analysis exists in the TCGA database was also included. To obtain these data we created a mapping of tumor types to corresponding normal tissues. For example, pancreatic cancer data from the TCGA database would be annotated with pancreas tissue from GTEX. In some cases, the mapping was approximate due to the lack of a clear tissue of origin for the tumor type. For example, the glioblastomas expression data were mapped from all tissues annotated as brain in GTEX.

Genes overexpressed in particular tissues, are likely to be good aCAR targets. Conversely, genes with even expression across all tissues are likely to be better iCAR targets.

A gene was defined as "universally expressed" if it met the following criteria: (i) the mean expression across tissues was greater than 10 RPKM (Reads Per Kilobase of transcript per Million mapped reads). (ii) The tissues with the least expression had an RPKM greater than 1. (iii) The ratio of the standard deviation in median RPKM across tissues compared to the mean RPKM was less than 1. Only **1,092** genes were annotated as universally expressed out of the **10,322** genes retrieved in the previous step.

### Annotation of Cell-Surface Proteins:

For a protein to be a good target for CAR-T mediated therapeutics, a portion of it needs to be expressed on the surface of the cell. We used several databases to help identify cell-surface proteins. The first database is the Human Protein Atlas, an analysis derived from 24,028 antibodies used to analyze 32 different tissues (Uhlen et al., 2015). The second database is the Cell Surface Protein Atlas, a mass-spectrometry based database of N-glycosylated cell-surface proteins (Bausch-Fluck et al., 2015). The third database is a recently published analysis using high-throughput immunofluorescence and automated image analysis to identify subcellular localization of proteins, including cell surface expression (Thul et al., 2017). Each SNP was annotated with the number of databases in which a protein appeared. Proteins encoded by genes in all three databases were said to be expressed at the cell-surface with "high confidence", two databases with "medium confidence" and one database with "low confidence". As shown in Table 3, **3,359** out of **10,322** genes had any evidence of membrane expression as described above.

**Table 3. Distribution of genes based on evidence of membrane expression**

| # databases with gene | Classification | Number of genes |
|---|---|---|
| 0 | Not membrane | 6963 |
| 1 | Low-confidence | 2904 |
| 2 | Medium-confidence | 408 |
| 3 | High-confidence | 47 |

Although the SNPs were annotated according to the above databases, the candidates were selected only based on the UniProt annotation as described below.

### Impact of allele on protein function:

For an iCAR to effectively recognize only cancer cells that have lost one allele of a membrane protein, the protein's structure ought to be sufficiently different based on which allele is encoded. The SIFT (Sorting Intolerant From Tolerant) algorithm attempts to predict whether a protein variant will have an effect on the protein structure, and therefore function (Ng and Henikoff, 2003). The score can range from 0 (deleterious) to 1 (benign). SIFT scores (version sift5.2.2) were included for every SNP for which a score was available.

### Classification of allele falling in the extracellular portion of the protein:

For an iCAR to recognize an allele, the allele must fall on the extracellular portion of the protein. For each SNP, the position of the amino acid affected in the consensus translation was extracted and compared to domains annotated as extracellular from the UniProt database, that was downloaded from www.uniprot.org/downloads. Many false negatives are possible due to a lack of characterization of the domains of all proteins. A total of **4577** SNPs in **1146** genes were annotated as extracellular.

### Proportion of tumors undergoing LOH

A good iCAR target would be a SNP that undergoes loss of heterozygosity (LOH) in a large fraction of tumors. Segments copy number files were downloaded from the cbio portal of the TCGA database http://www.cbioportal.org (Cerami et al., 2012, Gao et al., 2013). The proportion of tumors out of 32 tumors in the TCGA database undergoing LOH for each gene was determined as described below in more detail for the HLA genes.

### Example 2. Loss of Heterozygosity of HLA class-I proteins

HLA class-I genes were chosen as the first set of potential iCAR targets due to their already known characteristics: cell-surface proteins expressed from both alleles, a wide tissue distribution, a high level of polymorphism, and documented LOH in tumors as a mechanism of tumor escape. Hence, we started the analysis by determining the rate at which HLA class-I proteins are lost in various tumor types. We analyzed these copy number profiles for the presence of loss-of-heterozygosity at the genomic loci of HLA-A, B and C, listed in **Table 4.**

**Table 4. HLA-I genomic loci**

| Gene | Protein | Chromosome | Start Position | End Position |
|---|---|---|---|---|
| HLA-A | HLA-A | 6 | 29941260 | 29945884 |
| HLA-B | HLA-B | 6 | 31353872 | 31357188 |
| HLA-C | HLA-C | 6 | 31268749 | 31272130 |

SNP arrays data, across thousands of tumor samples, publicly available from the TCGA, can serve as a source for copy number calculation and was used to predict HLA LOH frequency across all tumor types available on the public NIH TCGA data portal (https://gdc.cancer.gov/).

In order to determine if the LOH calls are robust to changes in genomic position, we tested the LOH pattern of the genes located upstream (HLA-G, **Fig. 3A**) and downstream to HLA-A (ZNRD1, **Fig. 3C**) and concluded that all genes show the same pattern of LOH as HLA-A (**Fig. 3B**)

As all HLA class-I genes (HLA-A, B, C) are chromosomally located close to one another in the major histocompatibility locus (MHC) on the short arm of chromosome 6, as expected, they were all found to exhibit the same pattern and frequency of LOH across tumor types (**Fig. 4A****, and** **4B** for HLA-B and C, respectively).

Based on the above, and as shown in **Fig. 4**, we concluded that HLA class-I region LOH is a common event in many tumors, however and the percentage of LOH varies between tumor types. Therefore, HLA genes are good candidates for iCAR targets.

### Example 3. Immunocytochemical verification of LOH and specificity of allele specific antibodies.

Several pairs of preserved and lost allelic variants identified in different tumors are selected and their polypeptide products will serve for the generation of variant-specific mAbs. The discriminatory power of candidate mAbs are assayed by double staining and flow cytometry experiments or immunohistochemistry, as follows:

### IHC protocol

### Allele-specific anti-HLA_antibodies:

| Antibody- | Manufacturer |
|---|---|
| Anti-human HLA-A2 APC (BB7.2) | eBiosciences |
| Anti-human HLA-A2 PE-cy7 (BB7.2) | eBiosciences |
| Anti-human HLA-A3 FITC (GAP A3) | eBiosciences |
| Anti-human HLA-A3 PE (GAP A3) | eBiosciences |
| mouse anti-human HLA-B7-PE (BB7.1) | Millipore |
| HLA-A2 antibody (BB7.2) | Novus |
| HLA B7 antibody (BB7.1) | Novus |
| Mouse anti-human HLA-B27-FITC (HLA.ABC.m3) | Millipore |

### Frozen tissues samples -

Frozen tissues are often fixed in a formalin-based solution, and embedded in OCT (Optimal Cutting Temperature compound), that enables cryosectioning of the sample. Tissues in OCT are kept frozen at -80°C. Frozen blocks are removed from -80°C prior to sectioning, equilibrated in cryostat chamber, and cut to thin sections (often 5-15µm thick). Sections are mounted on a histological slide. Slides can be stored at -20°C to -80°C. Prior to IHC staining ,slides are thawed at room temperature (RT) for 10-20min

### Paraffin-embedded tissues -

Tissues are embedded in a Formaldehyde Fixative Solution. Prior to addition of the paraffin wax, tissues are dehydrated by gradual immersion in increasing concentrations of ethanol (70%, 90%, 100%) and xylene for specific times and durations at RT. Then, the tissues are embedded in paraffin wax.

The paraffin-embedded tissues are cut in a microtome to a 5-15µm thick sections, floated in a 56°C water bath, and mounted on a histological slide. Slides can be kept at RT.
Prior to IHC staining, paraffin-embedded sections require a rehydration step -
REHYDRATION - sections are rehydrated by immersion in xylene (2 X 10min), followed by decreasing concentrations of ethanol - 100% X2, each for 10min
95% ethanol - 5min
70% ethanol - 5min
50% ethanol - 5min
Rinsing in dH2O

### Immunofluorescence detection:

1. Rehydrate slides in wash buffer (PBSX1) for 10min. Drain the wash buffer
2. Perform antigen retrieval - if needed (heat-induced antigen retrieval or enzymatic retrieval)
3. For intracellular antigens, perform permeabilization - incubate the slides in 0.1% triton X-100 in PBSX1 for 10mm at RT.
4. BLOCKING - Block the tissue in blocking buffer for 30 min. at RT. Blocking buffer depends on the detection method, usually 5% animal serum in PBSX1, or 1% BSA in PBSX1
5. PRIMARY ANTIBODY - Dilute primary antibody in incubation buffer (i.e. 1% BSA, 1% donkey serum in PBS, other incubation buffers can also be used), according to antibody manufacturer instructions. Incubate the tissue in diluted primary antibody at 4°C overnight. The primary antibody may be a monoclonal anti-HLA-A, anti-HLA-B or anti-FILA-C allele-specific antibody as detailed above.
   If a conjugated primary antibody is used, protect from light, and proceed to step 8
   As a negative control, incubate the tissue with incubation buffer only, with no primary antibody
   Also, perform isotype matched control of the monoclonal antibody used in the experiment
6. WASH - wash slides in wash buffer - 3 X 5-15 min.
7. SECONDARY ANTIBODY - Dilute secondary antibody in incubation buffer according to antibody manufacturer instructions. Incubate the tissue in diluted secondary antibody for 30-60 min at RT. Protect from light
8. WASH - wash slides in wash buffer - 3 × 5-15 min.
9. DAPI staining - Dilute DAPI incubation buffer (~300nM - 3µM). Add 300µl of DAPI solution to each section. Incubate at RT for 5-10min.
10. WASH - wash slide once with Xl PBS
11. Mount with an antifade mounting media
12. Keep slides protected from light
13. Visualize slides using a fluorescence microscope

### Chromogenic detection:

1. Rehydrate slides in wash buffer (PBSX1) for 10min. Drain the wash buffer
2. Perform antigen retrieval - if needed- see above
3. For HRP reagents, block endogenous peroxidase activity with 3.0% hydrogen peroxide in methanol for at least 15min
4. Wash the sections by immersing them in dH₂O for 5min
5. For intracellular antigens, perform permeabilization - incubate the slides in 0.1 % triton X-100 in PBSX1 for 10min at RT.
6. BLOCKING - Block the tissue in blocking buffer for 30 min. at RT. Blocking buffer depends on the detection method, usually 5% animal serum in PBSX1, or 1% BSA in PBSX1
7. PRIMARY ANTIBODY - Dilute primary antibody in incubation buffer (i.e. 1% BSA, 1% donkey serum in PBS, other incubation buffers can also be used), according to antibody manufacturer instructions. Incubate the tissue in diluted primary antibody at 4°C overnight
8. WASH - wash slides in wash buffer - 3 × 5-15 min.
9. SECONDARY ANTIBODY - Incubate the tissue in HRP-conjugated secondary antibody for 30-60 min at RT.
10. WASH - wash slides in wash buffer 3 × 5-15mm.
11. Add ABC-HRP reagent according to manufacturer guidelines. Incubate at RT for 60min.
12. Prepare DAB solution (or other chromogen) according to manufacturer guidelines, and apply to tissue sections. The chromogenic reaction turns the epitope sites brown (usually few seconds - 10 minutes). Proceed to the next step when the intensity of the signal is appropriate for imaging
13. WASH - wash slides in wash buffer - 3 × 5-15 min.
14. Wash slides in dH₂O - 2 × 5-15min.
15. Nuclei staining - add Hematoxylin solution. Incubate at RT for 5min.
16. Dehydrate tissue sections -
   95% ethanol - 2 × 2min.
   100% ethanol - 2 × 2min.
   Xylene - 2 × 2min.
17. Mount with an antifade mounting media
18. Visualize slides using a bright-field illumination

### Example 4. CAR-T construction

The purpose of the study is to create a synthetic receptor which will inhibit the on-target 'off-tumor' effect of CAR-T therapy. To that extent a library of CAR constructs composed of activating and inhibitory CARs will be established.

The first set of constructs will include an inhibitory CAR directed at HLA- type I sequence (for example, HLA-A2) and an activating CAR directed at tumor antigen (for example CD19). The next set of constructs will include CAR sequences directed at target antigens identified by our bioinformatics analysis. Target candidates will be prioritized according to set forth criteria (for example target expression pattern, target expression level, antigenicity and more).

For the iCAR constructs, we will fuse the transmembrane and intracellular domains up to the first annotated extracellular domain of PD-1 (amino acid 145-288) or CTLA4 (amino acids 161-223), downstream to HLA-A2 scFV. For iCAR detection and sorting, a reporter gene (i. e. GFP, DsRED, RFP, mCherry etc.) will be integrated downstream to the iCAR sequence via IRES sequences or 2A sequences.

The HLA-A2 scFv is cloned and constructed from hybridomas expressing any one of the antibodies listed above in Example 2.

For the aCAR construct, CD19 scFV will be fused to 2^{nd} generation (CD8 or CD28 hinge, CD28 transmembrane, CD28 or 41BB co-stimulation 1 and CD3ζ) or 3^{rd} generation CAR (CD8 or CD28 hinge, CD28 transmembrane, CD28 and 41BB and CD3ζ) for aCAR detection and sorting a reporter gene (i. e. GFP, DsRed, RFP, mCherry etc.) will be integrated downstream to the aCAR sequence via IRES sequences or 2A sequences.

Both aCAR and iCAR sequences will be cloned into retrovirus or lentivirus transfer vectors and will be then used for viral particle production using appropriate packaging cells like for example HEK-293T.

Jurkat, Jurkat-NFAT-Luciferase and activated T cells derived from healthy donors will be transduced with aCAR, iCAR or both, at different multiplicity of infection (MOI). FACS selection based on reporter gene expression will be used for sorting and selection of cell population expressing different level of aCAR, iCAR or both.

### Preparation of target cells -

An in vitro recombinant system will be established for testing the functionality of the iCAR constructs in inhibiting the activity of the aCAR towards the off-target cells. For this purpose, target cells expressing the aCAR epitope, iCAR epitope or both will be produced. The recombinant cells expressing the aCAR epitope will represent the on-target 'on-tumor' cells while the cells expressing both aCAR and iCAR epitopes would represent the on target 'off-tumor' healthy cells.

As our first iCAR/aCAR set will be HLA-A2, CD19 respectively, recombinant cells expressing HLA-A2, CD19 or both will be produced, by transfecting cell line (i.e. Hela, Hela-Luciferase or Raji) with expression vector coding for these genes. For detection of recombinant CD19 and HLA A-2expression, both genes will be fused to a protein tag (i.e. HA or Flag or Myc etc).

### Assays-

The inhibitory effect of the iCAR will be tested both in-vitro and in-vivo.

In the in-vitro assays we will focus on measuring cytokine secretion and cytotoxicity effects, while in-vivo, we will evaluate the iCAR inhibition and protection to on-target off tumor xenografts. We will limit T cells lacking iCAR from contaminating the results by sorting T cells to be 1CAR/aCAR double positive using reporter genes. As a negative control for the iCAR blocking activity we may use mock transfected CAR lacking the scFv domain.

### In vitro assays-

Luciferase cytotoxic assay - Recombinant target cells (T) will be engineered to express firefly luciferase. In-vitro luciferase assay will be performed according to the Bright-Glo Luciferase assay (Promega). Transduced effector (E) T cells (transduced with both iCAR and aCAR or aCAR or mock CAR) will be incubated for 24-48 hrs with recombinant cells expressing HLA-A2, CD19 or both in different effector to target ratios. Cell killing will be quantified with the Bright-Glo Luciferase system.

We may optimize the 'off-tumor' cytotoxicity by sorting transduced T cells population according to iCAR/aCAR expression level or by selecting sub population of recombinant target cells according to their CD19 or HLA-A2 expression level.

To test whether the iCAR transduced T cells can discriminate between the 'on-tumor' and 'off-tumor' cells in vitro, we will test the killing effect of transduced T cells incubated with a mix of 'on-tumor' and 'off-tumor' cells at a ratio of 1:1. The on tumor recombinant cells will be distinguished from the 'off-tumor' recombinant cells by Luciferase expression (only one cell population will be engineered to express the luciferase gene at a time). Killing will be quantified after 24-48 hrs of co-incubation using the Bright-Glo Luciferase assay (Promega).

### Caspase 3-detection of CTL induced apoptosis by an antibody to activated cleaved caspase 3.

One of the pathways by which CTL kill target cells is by inducing apoptosis through the Fas ligand. The CASP3 protein is a member of the cysteine-aspartic acid protease (caspase) family. Sequential activation of caspases plays a significant role in the execution-phase of cell apoptosis. Cleavage of pro-caspase 3 to caspase 3 results in conformational change and expression of catalytic activity. The cleaved activated form of caspase 3 can be recognized specifically by a monoclonal antibody.

Transduced T cells will be incubated with either 'on-tumor' or 'off-tumor' recombinant cells, previously labeled with CFSE, for 2-4 hrs. Target cells apoptosis will be quantified by flow cytometry. Cells will be permeabilized and fixed by an inside staining kit (Miltenyi) and stained with an antibody for activated caspase 3 (BD bioscience).

### Time lapse micros CTL-

Target cells will be labeled with a reporter gene (for example- mCherry). Transduced T cells will be incubated with either 'on-tumor' or 'off-tumor' cells for up to 5 days. Time lapse microscopy will be used to visualize killing. Alternatively, flow cytometry analysis using viable cell number staining and CountBright beads (Invitrogen) for determining target cells number at end-point time will be conducted.

In order to check if the aCAR/iCAR transduced T cells can discern targets in vitro, we will label each recombinant target cells ('on-tumor' or 'off-tumor') with a different reporter protein (for example GFP and mCherry). Transduced T cells (Effector cells) will be co-incubated with the recombinant cells (target cells) at a 1:1 ratio of E/T. We will then follow cell fate by microscopy imaging.

### Cytokine release-

Transduced T cells will be incubated with the recombinant target cells and cytokine production (IL2 and or INFγ) will be quantified either by measuring cytokine secretion in cell culture supernatant according to BioLegend's ELISA MAX^{™} Deluxe Set kit, or by flow cytometry analysis of the percentage of T cells producing cytokines. For the flow cytometry analysis, we will use a Golgi stop to prevent the secretion of the cytokines. Following a 6 and 18-24 hrs incubation of the transduced T cells with target cells, T cells will be permed and fixed by an inside staining kit (Miltenyi) and stained with antibodies for the T cell markers (CD3 and CD8) and for the cytokines IL2 and INFγ.

### Staining for CD1 07a

Degranulating of T cells can be identified by the surface expression of CD107a, a lysosomal associated membrane protein (LAMP-1). Surface expression of LAMP-1 has been shown to correlate with CD8 T cell cytotoxicity. This molecule is located on the luminal side of lysosomes. Upon activation, CD107a is transferred to the cell membrane surface of activated lymphocytes. CD107a is expressed on the cell surface transiently and is rapidly re-internalized via the endocytic pathway. Therefore, CD107a detection is maximized by antibody staining during cell stimulation and by the addition of monensin (to prevent acidification and subsequent degradation of endocytosed CD107a antibody complexes).

We will incubate the transduced T cells with the target cells for 6-24 hrs in the presence of monensin and will follow CD107a expression on the CD8 T cells by flow cytometry using conjugated antibodies against the T cell surface markers (CD3,CD8) and a conjugated antibody for CD107a.

### In vivo

NOD/SCID/γc- mice will be inoculated intravenously with tumor cells. One possibility of tumor cells could be the CD19 positive NALM 6 (ATCC, human B-ALL cell line) cells that will be engineered to express firefly luciferase. In addition, for establishment of 'on-target' 'off-tumor' cells, NALM 6 will also be engineered to express the iCAR epitope (for example HLA-A2) thereby representing the healthy cells. Mice will be divided into study groups, one group will be injected with the NALM 6 cells while the other will be injected with the NALM-6 expressing the iCAR epitope. Several days later, mice will be infused intravenously with T cells transduced with aCAR, aCAR/iCAR and a control group of untransduced T cells or no T cells. Mice will be sacrificed and tumor burden will be quantified according to total flux.
To test whether the T cells expressing the iCAR construct could discriminate between the target cells and off target cells in vivo within the same organism, we will inject mice with a 1:1 mixture of the 'on-tumor'/'off-tumor NALM-6 cells, followed by injection of transduced T cells expressing either the aCAR alone or both aCAR and iCAR. Upon sacrifice of the mice the presence of the 'on-tumor' and 'off-tumor cells in the spleen and bone marrow will be analyzed by flow cytometry for the two markers, CD19 and the iCAR epitope.

### APPENDIX.

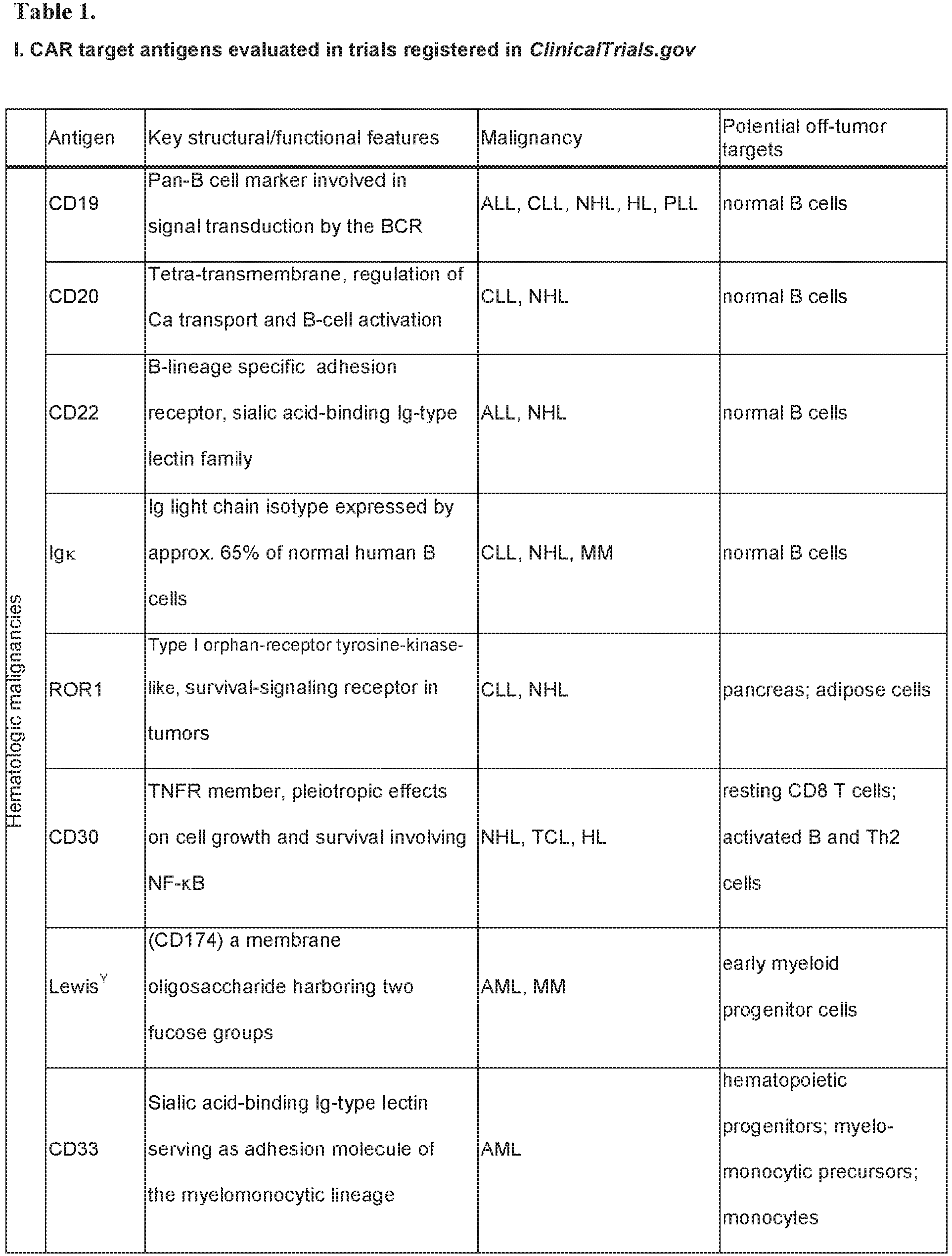

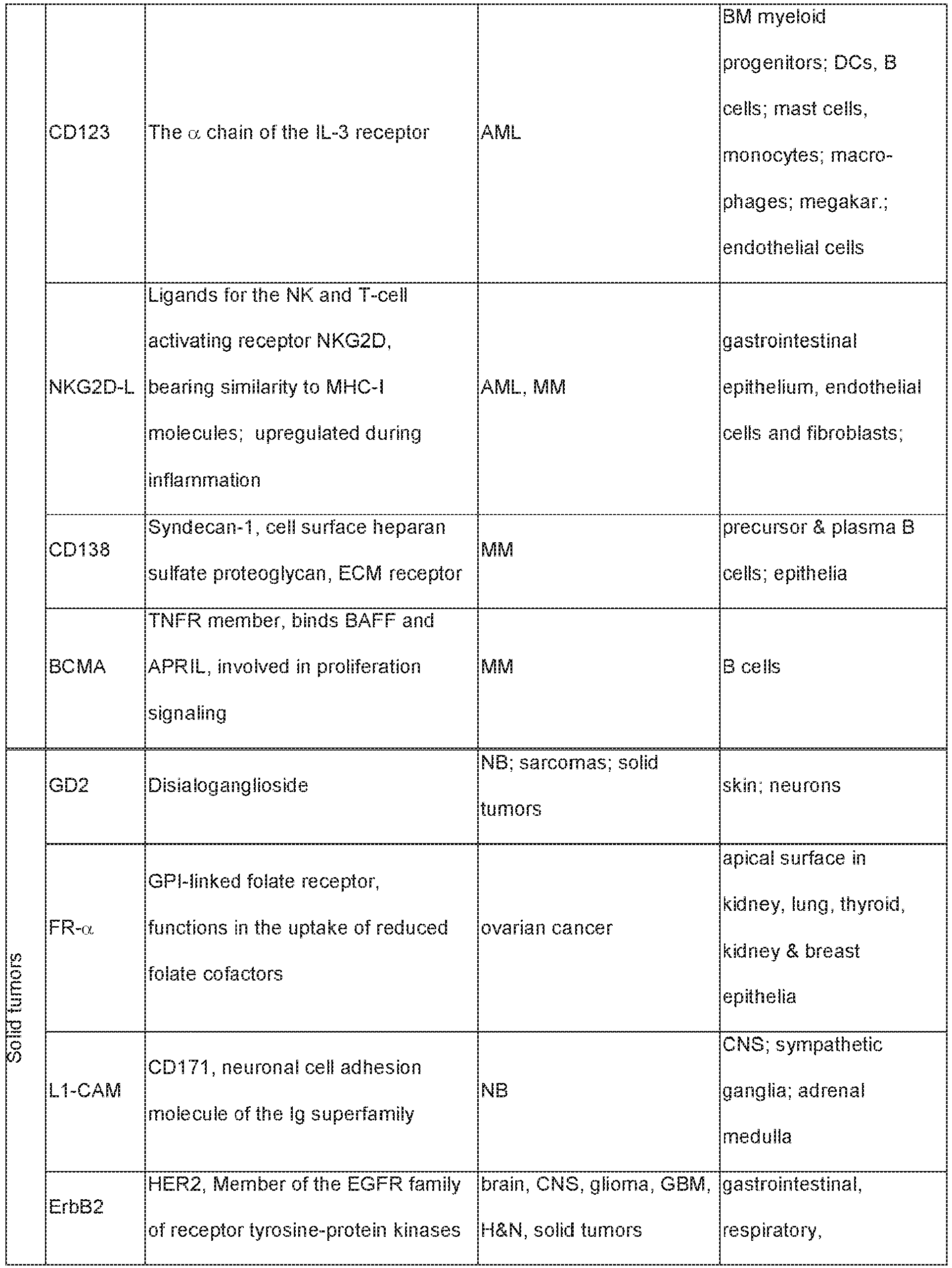

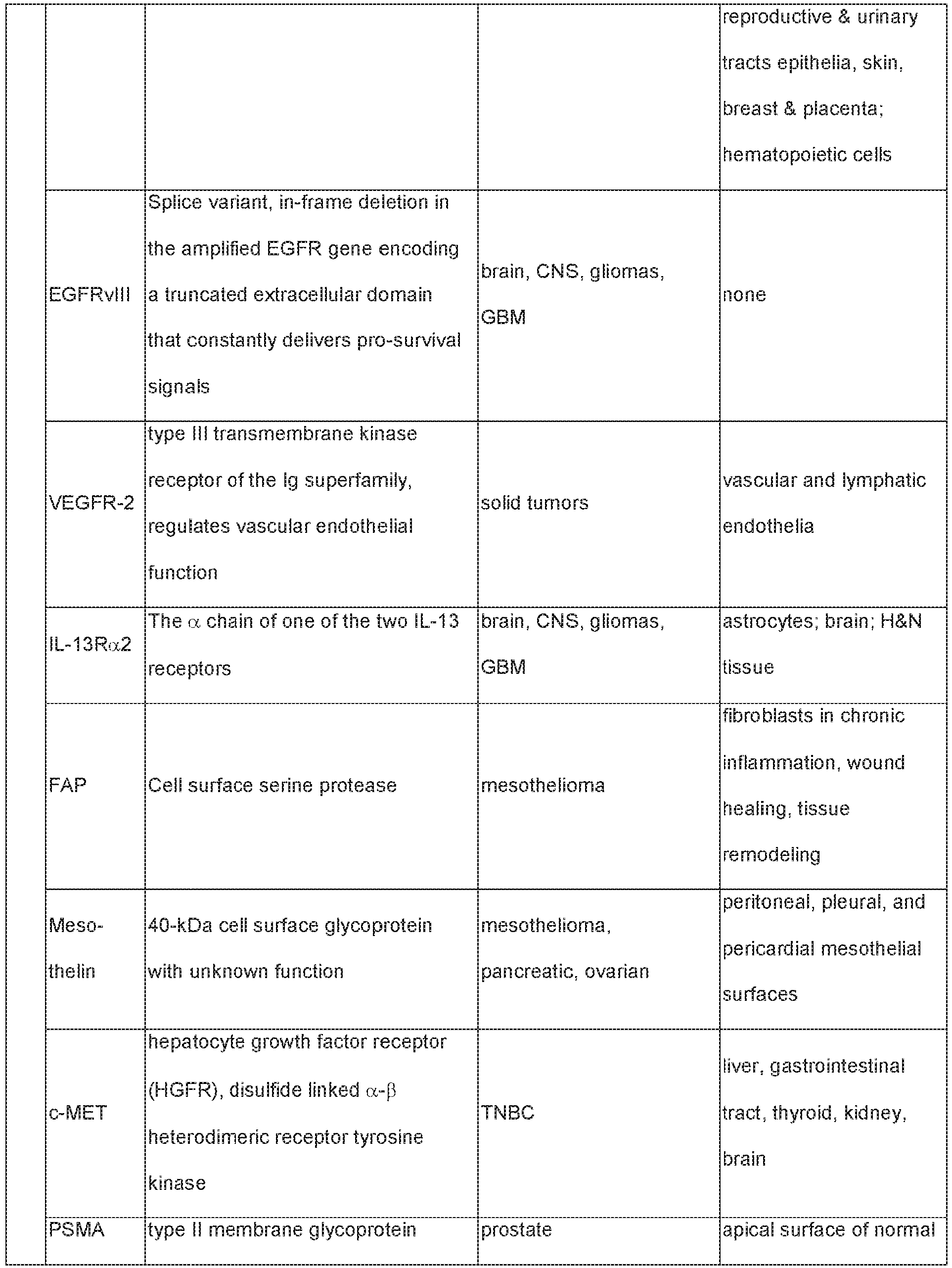

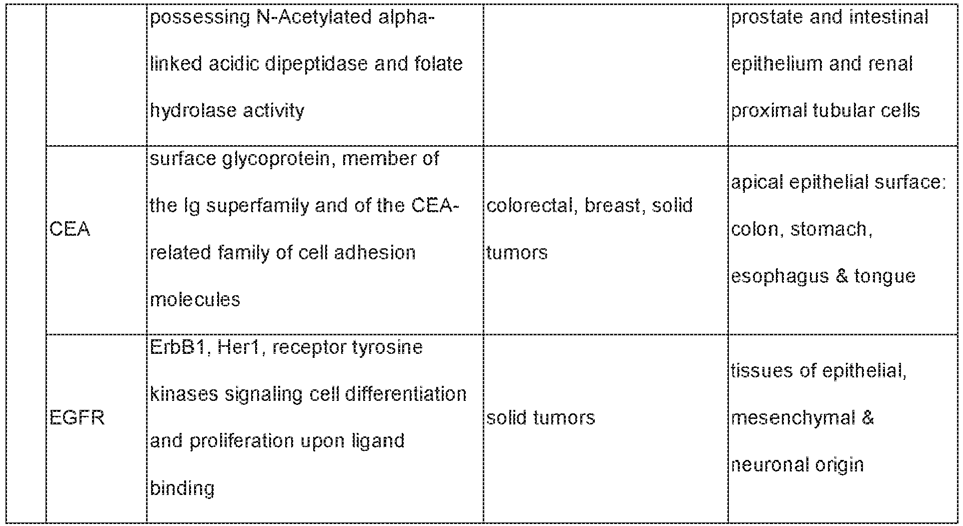

### II. Other CAR target antigens

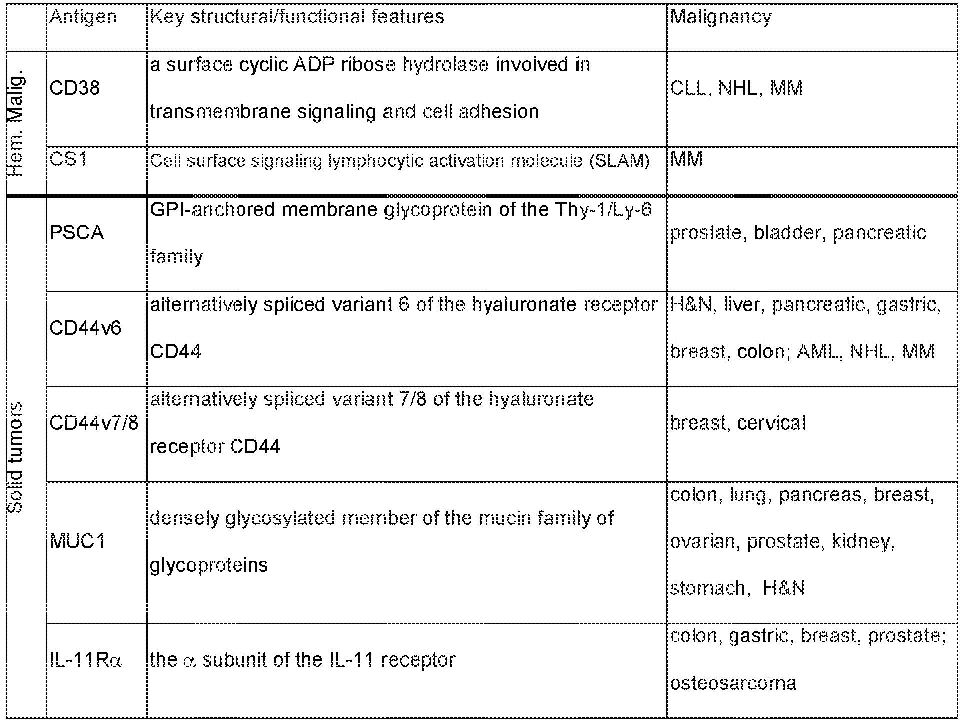

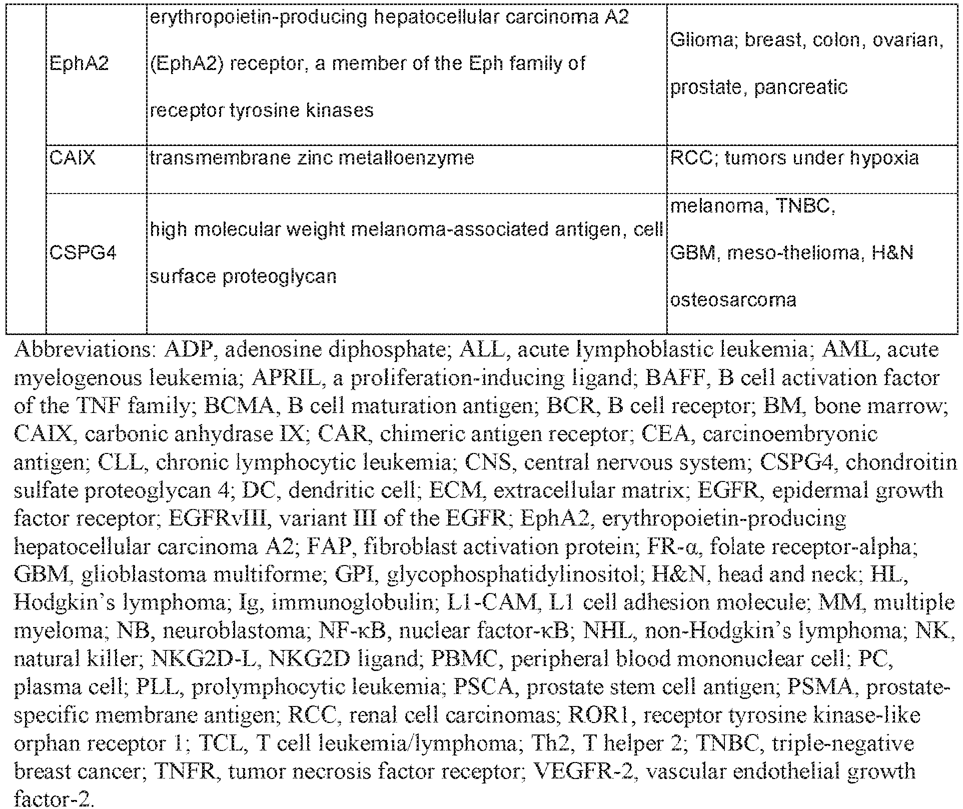

### REFERENCES

Abecasis, G.R., Altshuler, D., Auton, A., Brooks, L.D., Durbin, R.M., Gibbs, R.A., Hurles, M.E., and McVean, G.A. (2010). A map of human genome variation from population-scale sequencing. Nature 467, 1061-1073.
Abeyweera, T.P., Merino, E., and Huse, M. (2011). Inhibitory signaling blocks activating receptor clustering and induces cytoskeletal retraction in natural killer cells. J. Cell Biol. 192, 675-690.
Auton, A., Abecasis, G.R., Altshuler, D.M., Durbin, R.M., Bentley, D.R., Chakravarti, A., Clark, A.G., Donnelly, P., Eichler, E.E., Flicek, P., et al. (2015). A global reference for human genetic variation. Nature 526, 68-74.
Barbas, Carlos F., Dennis R. Burton, Jamie K. Scott, G. J. S. 2004. Phage display : a laboratory manual,. Cold Spring Harbor Laboratory Press.
Bausch-Fluck, D., Hofmann, A., Bock, T., Frei, A.P., Cerciello, F., Jacobs, A., Moest, H., Omasits, U., Gundry, R.L., Yoon, C., et al. (2015). A mass spectrometric-derived cell surface protein atlas. PLoS One 10.
Bayle, J.H., Grimley, J.S., Stankunas, K., Gestwicki, J.E., Wandless, T.J., and Crabtree, G.R. (2006). Rapamycin analogs with differential binding specificity permit orthogonal control of protein activity. Chem. Biol. 13, 99-107.
Bergbold, N., and Lemberg, M.K. (2013). Emerging role of rhomboid family proteins in mammalian biology and disease. Biochim. Biophys. Acta 1828, 2840-2848.
Blankenstem, T., Leisegang, M., Uckert, W., and Schreiber, H. (2015). Targeting cancer-specific mutations by T cell receptor gene therapy. Curr. Opin. Immunol. 33, 112-119.
Boczkowski, D., S. K. Nair, J. H. Nam, H. K. Lyerly, and E. Gilboa. 2000. Induction of tumor immunity and cytotoxic T lymphocyte responses using dendritic cells transfected with messenger RNA amplified from tumor cells. Cancer Res 60: 1028-34.Barrett, M.T., Sanchez, C.A., Prevo, Burrell, R.A., McGranahan, N., Bartek, J., and Swanton, C. (2013). The causes and consequences of genetic heterogeneity in cancer evolution. Nature 501, 338-345.
Van Buuren, M.M., Calis, J.J.A., and Schumacher, T.N.M. (2014). High sensitivity of cancer exome-based CD8 T cell neo-antigen identification. Oncoimmunology 3.
Caescu, C.I., Jeschke, G.R., and Turk, B.E. (2009). Active-site determinants of substrate recognition by the metalloproteinases TACE and ADAM10. Biochem. J. 424, 79-88.
Carney, W.P., Petit, D., Hamer, P., Der, C.J., Finkel, T., Cooper, G.M., Lefebvre, M., Mobtaker, H., Delellis, R., and Tischler, A.S. (1986). Monoclonal antibody specific for an activated RAS protein. Proc. Natl. Acad. Sci. U. S. A. 83, 7485-7489.
Cerami E, et al. The cbio cancer genomics portal: An open platform for exploring multidimensional cancer genomics data. Cancer discovery. 2012;2:401-404.
Chao, G., W. L. Lau, B. J. Hackel, S. L. Sazinsky, S. M. Lippow, and K. D. Wittrup. 2006. Isolating and engineering human antibodies using yeast surface display. Nat. Protoc. 1*.*
Chess, A. (2012). Mechanisms and consequences of widespread random monoallelic expression. Nat. Rev. Genet. 13, 421-428.
Chicaybam, L., and Bonamino, MH. (2014). Abstract 2797: Construction and validation of an activating and inhibitory chimeric antigen receptor (CAR) system. Cancer Res. 74, 2797-2797.
Chicaybam, L., and Bonamino, M.H. (2015). Abstract 3156: Construction and validation of an activating and inhibitory chimeric antigen receptor (CAR) system. Cancer Res. 75, 3156-3156.
Consortium GT. Human genomics. The genotype-tissue expression (gtex) pilot analysis: Multitissue gene regulation in humans. Science. 2015;348:648-660.
Da Cunha, J.P.C., Galante, P.A.F., De Souza, J.E., De Souza, R.F., Carvalho, P.M., Ohara, D.T., Moura, R.P., Oba-Shinja, S.M., Marie, S.K.N., Silva Jr., W.A., et al. (2009). Bioinformatics construction of the human cell surfaceome. Proc. Natl. Acad. Sci. U. S. A. 106, 16752-16757.
Devilee, P., Cleton-Jansen, A.-M., and Cornelisse, C.J. (2001). Ever since Knudson. Trends Genet. 17, 569-573.
Dotti, G., Gottschalk, S., Savoldo, B., and Brenner, M.K. (2014). Design and development of therapies using chimeric antigen receptor-expressing T cells. Immunol. Rev. 257, 107-126.
Ebsen, H., Schröder, A., Kabelitz, D., and Janssen, O. (2013). Differential surface expression of ADAM10 and ADAM17 on human T lymphocytes and tumor cells. PLoS One 8, e76853.
Eriksson, M., Leitz, G., Fällman, E., Axner, O., Ryan, J.C., Nakamura, M.C., and Sentman, C.L. (1999). Inhibitory receptors alter natural killer cell interactions with target cells yet allow simultaneous killing of susceptible targets. J. Exp. Med. 190, 1005-1012.
Fedorov, V.D., Themeli, M., and Sadelain, M. (2013a). PD-1- and CTLA-4-based inhibitory chimeric antigen receptors (iCARs) divert off-target immunotherapy responses. Sci. Transl. Med. 5, 215ra172.
Fedorov, V.D., Themeli, M., and Sadelain, M. (2013b). PD-1- and CTLA-4-based inhibitory chimeric antigen receptors (iCARs) divert off-target immunotherapy responses. In Science Translational Medicine, (Affiliation: Center for Cell Engineering, Memorial Sloan-Kettering Cancer Center (MSKCC), New York, NY 10065, United States; Affiliation: Tri-Institutional MSTP Program (MSKCC, Rockefeller University, Weill-Cornell Medical College), New York, NY 10065, Un),.
Feenstra, M., Veltkamp, M., van Kuik, J., Wiertsema, S., Slootweg, P., van den Tweel, J., de Weger, R., and Tilanus, M. (1999). HLA class I expression and chromosomal deletions at 6p and 15q in head and neck squamous cell carcinomas. Tissue Antigens 54, 235-245.
Gao J. et al, Integrative analysis of complex cancer genomics and clinical profiles using the cBio Portal. Sci Signal. 2013 2;6(269)
Gill, S., and June, C.H. (2015). Going viral: chimeric antigen receptor T-cell therapy for hematological malignancies. Immunol. Rev. 263, 68-89.
Gordon, W.R., Zimmerman, B., He, L., Miles, L.J., Huang, J., Tiyanont, K., McArthur, D.G., Aster, J.C., Perrimon, N., Loparo, J.J., et al. (2015). Mechanical Allostery: Evidence for a Force Requirement in the Proteolytic Activation of Notch. Dev. Cell 33, 729-736.
Graef, I.A., Holsinger, L.J., Diver, S., Schreiber, S.L., and Crabtree, G.R. (1997). Proximity and orientation underlie signaling by the non-receptor tyrosine kinase ZAP70. EMBO J. 16, 5618-5628.
Gross, G., and Eshhar, Z. (2016a). Therapeutic Potential of T-Cell Chimeric Antigen Receptors in Cancer Treatment: Counteracting Off-Tumor Toxicities for Safe CAR T-Cell Therapy. Annu. Rev. Pharmacol. Toxicol. 2016. 56:59-83.
Gross, G., and Eshhar, Z. (2016b). Therapeutic Potential of T Cell Chimeric Antigen Receptors (CARs) in Cancer Treatment: Counteracting Off-Tumor Toxicities for Safe CAR T Cell Therapy. Annu. Rev. Pharmacol. Toxicol. 56, 59-83.
Gross, G., Waks, T., and Eshhar, Z. (1989). Expression of immunoglobulin-T-cell receptor chimeric molecules as functional receptors with antibody-type specificity. Proc Natl Acad Sci U S A 86, 10024-10028.
Haapasalo, A., and Kovacs, D.M. (2011). The many substrates of presenilin/γ-secretase. J. Alzheimers. Dis. 25, 3-28.
Hanes, J., and A. Plückthun. 1997. In vitro selection and evolution of functional proteins by using ribosome display. Proc. Natl. Acad. Sci. U. S. A. 94.
Heemskerk, B., Kvistborg, P., and Schumacher, T.N.M. (2013). The cancer antigenome. EMBO J. 32, 194-203.
Hemming, M.L., Elias, J.E., Gygi, S.P., and Selkoe, D.J. (2009). Identification of beta-secretase (BACE1) substrates using quantitative proteomics. PLoS One 4, e8477.
Huse, M., Catherine Milanoski, S., and Abeyweera, T.P. (2013). Building tolerance by dismantling synapses: inhibitory receptor signaling in natural killer cells. Immunol. Rev. 251, 143-153.
Jiménez, P., Cantón, J., Collado, A., Cabrera, T., Serrano, A., Real, L.M., Garcia, A., Ruiz-Cabello, F., and Garrido, F. (1999). Chromosome loss is the most frequent mechanism contributing to HLA haplotype loss in human tumors. Int. J. Cancer 83, 91-97.
Klebanoff, C.A., Rosenberg, S.A., and Restifo, N. P. (2016). Prospects for gene-engineered T cell immunotherapy for solid cancers. Nat. Med. 22, 26-36.
Kloss, C.C., Condomines, M., Cartellieri, M., Bachmann, M., and Sadelain, M. (2013). Combinatorial antigen recognition with balanced signaling promotes selective tumor eradication by engineered T cells. Nat. Biotechnol. 31, 71-75.
Knudson Jr., A.G. (1971). Mutation and cancer: statistical study of retinoblastoma. Proc. Natl. Acad. Sci. U. S. A. 68, 820-823.
Lanitis, E., Poussin, M., Klattenhoff, A.W., Song, D., Sandaltzopoulos, K., June, C.H., and Powell Jr, D.J. (2013). Chimeric antigen receptor T cells with dissociated signaling domains exhibit focused anti-tumor activity with reduced potential for toxicity. Cancer Immunol. Res. 1, 10.1158/2326-6066.CIR - 13-0008.
Lawrence, M.S., Stojanov, P., Polak, P., Kryukov, G. V, Cibulskis, K., Sivachenko, A., Carter, S.L., Stewart, C., Mermel, C.H., Roberts, S.A., et al. (2013). Mutational heterogeneity in cancer and the search for new cancer-associated genes. Nature 499, 214-218.
Lee, A., Rana, B.K., Schiffer, H.H., Schork, N.J., Brann, M.R., Insel, P.A., and Weiner, D.M. (2003). Distribution analysis of nonsynonymous polymorphisms within the G-protein-coupled receptor gene family. Genomics 81, 245-248.
Lek M, et al., Exome Aggregation C. Analysis of protein-coding genetic variation in 60,706 humans. Nature. 2016;536:285-291.
Lengauer, C., Kinzler, K.W., and Vogelstein, B. (1998). Genetic instabilities in human cancers. Nature 396, 64-649.
Li, H., Yang, B., Xing, K., Yuan, N., Wang, B., Chen, Z., He, W., and Zhou, J. (2014). A preliminary study of the relationship between breast cancer metastasis and loss of heterozygosity by using exome sequencing. Sci. Rep. 4*.*
Liberles, S.D., Diver, S.T., Austin, D.J., and Schreiber, S.L. (1997). Inducible gene expression and protein translocation using nontoxic ligands identified by a mammalian three-hybrid screen. Proc. Natl. Acad. Sci. 94, 7825-7830.
Lindblad-Toh, K., Tanenbaum, D.M., Daly, M.J., Winchester, E., Lui, W.-O., Villapakkam, A., Stanton, S.E., Larsson, C., Hudson, T.J., Johnson, B E., et al. (2000). Loss-of-heterozygosity analysis of small-cell lung carcinomas using single-nucleotide polymorphism arrays. Nat. Biotechnol. 18, 1001-1005.
Lo, K.C., Bailey, D., Burkhardt, T., Gardina, P., Turpaz, Y., and Cowell, J.K. (2008). Comprehensive analysis of loss of heterozygosity events in glioblastoma using the 100K SNP mapping arrays and comparison with copy number abnormalities defined by BAC array comparative genomic hybridization. Genes Chromosom. Cancer 47, 221-237.
Long, E.O., Sik Kim, H., Liu, D., Peterson, M.E., and Rajagopalan, S. (2013). Controlling natural killer cell responses: Integration of signals for activation and inhibition. Annu. Rev. Immunol. 31, 227-258.
Maleno, I., López-Nevot, M.A., Cabrera, T., Salinero, J., and Garrido, F. (2002). Multiple mechanisms generate HLA class I altered phenotypes in laryngeal carcinomas: high frequency of HLA haplotype loss associated with loss of heterozygosity in chromosome region 6p21. Cancer Immunol. Immunother. 51, 389-396.
Maleno, I., Cabrera, C.M., Cabrera, T., Paco, L., López-Nevot, M.A., Collado, A., Ferrón, A., and Garrido, F. (2004). Distribution of HLA class I altered phenotypes in colorectal carcinomas: high frequency of HLA haplotype loss associated with loss of heterozygosity in chromosome region 6p21. Immunogenetics 56, 244-253.
Maleno, I., Romero, J.M., Cabrera, T., Paco, L., Aptsiauri, N., Cozar, J.M., Tallada, M., López-Nevot, M.A., and Garrido, F. (2006). LOH at 6p21.3 region and HLA class I altered phenotypes in bladder carcinomas. Immunogenetics 58, 503-510.
Maleno, I., Aptsiauri, N., Cabrera, T., Gallego, A., Paschen, A., López-Nevot, M.A., and Garrido, F. (2011). Frequent loss of heterozygosity in the β2-microglobulin region of chromosome 15 in primary human tumors. Immunogenetics 63, 65-71.
McGranahan, N., Burrell, R.A., Endesfelder, D., Novelli, M.R., and Swanton, C. (2012). Cancer chromosomal instability: Therapeutic and diagnostic challenges. EMBO Rep. 13, 528-538.
Morsut, L., Roybal, K.T., Xiong, X., Gordley, R.M., Coyle, S.M., Thomson, M., and Lim, W.A. (2016). Engineering Customized Cell Sensing and Response Behaviors Using Synthetic Notch Receptors. Cell 164, 780-791.
Ng PC, Henikoff S. Sift: Predicting amino acid changes that affect protein function. Nucleic acids research. 2003;31:3812-3814.
Nirschl, C.J., and Drake, C.G. (2013). Molecular pathways: Coexpression of immune checkpoint molecules: Signaling pathways and implications for cancer immunotherapy. Clin. Cancer Res. 19, 4917-4924,
O'Keefe, C., McDevitt, M.A., and Maciejewski, J.P. (2010). Copy neutral loss of heterozygosity: A novel chromosomal lesion in myeloid malignancies. Blood 115, 2731-2739.
Ohgaki, H., Dessen, P., Jourde, B., Horstmann, S., Nishikawa, T., Di Patre, P.-L., Burkhard, C., Schüler, D., Probst-Hensch, N.M., Maiorka, P C., et al. (2004). Genetic pathways to glioblastoma: a population-based study. Cancer Res. 64, 6892-6899.
Overwijk, W.W., Wang, E., Marincola, F.M., Rammensee, H.G., and Restifo, N.P. (2013). Mining the mutanome: developing highly personalized Immunotherapies based on mutational analysis of tumors. J. Immunother. Cancer 1, 11.
Rana, B.K., Shiina, T., and Insel, P.A. (2001). Genetic variations and polymorphisms of G protein-coupled receptors: functional and therapeutic implications. Annu. Rev. Pharmacol. Toxicol. 41, 593-624.
Rawson, R.B. (2013). The site-2 protease. Biochim. Biophys. Acta 1828, 2801-2807.
Rosenberg, S.A. (2014). Finding suitable targets is the major obstacle to cancer gene therapy. Cancer Gene Ther. 21, 45-47.
Rosenberg, S.A., and Restifo, N.P. (2015). Adoptive cell transfer as personalized immunotherapy for human cancer. Science 348, 62-68.
Roybal, K.T., Rupp, L.J., Morsut, L., Walker, W.J., McNally, K.A., Park, J.S., and Lim, W.A. (2016a). Precision Tumor Recognition by T Cells With Combinatorial Antigen-Sensing Circuits. Cell.
Roybal, K.T., Rupp, L.J., Morsut, L., Walker, W.J., McNally, K.A., Park, J.S., and Lim, W.A. (2016b). Precision Tumor Recognition by T Cells With Combinatorial Antigen-Sensing Circuits. Cell 164, 770-779.
Sathirapongsasuti, J.F., Lee, H., Horst, B.A.J., Brunner, G., Cochran, A.J., Binder, S., Quackenbush, J., and Nelson, S.F. (2011). Exome sequencing-based copy-number variation and loss of heterozygosity detection: ExomeCNV. Bioinformatics 27, 2648-2654.
Savage, P.A. (2014). Tumor antigenicity revealed. Trends Immunol. 35, 47-48.
Savova, V., Chun, S., Sohail, M., McCole, R.B., Witwicki, K., Gai, L., Lenz, T.L., Wu, C.-T., Sunyaev, S.R., and Gimelbrant, A.A. (2016). Genes with monoallelic expression contribute disproportionately to genetic diversity in humans. Nat. Genet. 48, 231-237.
Schumacher, T.N., and Schreiber, R.D. (2015). Neoantigens in cancer immunotherapy. Science (80-. ). 348, 69-74.
Sela-Culang, I., Y. Ofran, and B. Peters. 2015a. Antibody specific epitope prediction - Emergence of a new paradigm. Curr. Opin. Virol. 11*.*
Sela-Culang, I., S. Ashkenazi, B. Peters, and Y. Ofran. 2015b. PEASE: Predicting B-cell epitopes utilizing antibody sequence. Bioinformatics 31*.*
Skora, A.D., Douglass, J., Hwang, M.S., Tam, A.J., Blosser, R.L., Gabelli, S.B., Cao, J., Diaz, L.A., Papadopoulos, N., Kinzler, K.W., et al. (2015). Generation of MANA bodies specific to HLA-restricted epitopes encoded by somatically mutated genes. Proc. Natl. Acad. Sci. U. S. A. 112, 9967-9972.
Stark, M., and Hayward, N. (2007). Genome-wide loss of heterozygosity and copy number analysis in melanoma using high-density single-nucleotide polymorphism arrays. Cancer Res. 67, 2632-2642.
Stark, S.E., and Caton, A.J. (1991). Antibodies that are specific for a single amino acid interchange in a protein epitope use structurally distinct variable regions. J. Exp. Med. 174, 613-624.
Teo, S.M., Pawitan, Y., Ku, C.S., Chia, K.S., and Salim, A. (2012). Statistical challenges associated with detecting copy number variations with next-generation sequencing. Bioinformatics 28, 2711-2718.
Thul PJ, et al. A subcellular map of the human proteome. Science. 2017;356.
Treanor, B., Lanigan, P.M.P., Kumar, S., Dunsby, C., Munro, I., Auksorius, E., Culley, F.J., Purbhoo, M.A., Phillips, D., Neil, M.A.A., et al. (2006). Microclusters of inhibitory killer immunoglobulin-like receptor signaling at natural killer cell immunological synapses. J. Cell Biol. 174, 153-161.
Uhlen M, et al. Tissue-based map of the human proteome. Science. 2015;347:1260419.
Vogelstein, B., Fearon, E.R., Kern, S.E., Hamilton, S.R., Preisinger, A.C., Nakamura, Y., and White, R. (1989). Allelotype of colorectal carcinomas. Science (80-. ). 244, 207-211.
Vogelstein, B., Papadopoulos, N., Velculescu, V.E., Zhou, S., Diaz Jr., L.A., and Kinzler, K.W. (2013). Cancer genome landscapes. Science (80-. ). 340, 1546-1558.
Voss, M., Schröder, B., and Fluhrer, R. (2013). Mechanism, specificity, and physiology of signal peptide peptidase (SPP) and SPP-like proteases. Biochim. Biophys. Acta 1828, 2828-2839.
Vyas, Y.M., Mehta, K.M., Morgan, M., Maniar, H., Butros, L., Jung, S., Burkhardt, J.K., and Dupont, B. (2001). Spatial organization of signal transduction molecules in the NK cell immune synapses during MHC class I-regulated noncytolytic and cytolytic interactions. J. Immunol. 167, 4358-4367.
Wang, Z.C., Lin, M., Wei, L.-J., Li, C., Miron, A., Lodeiro, G., Harris, L., Ramaswamy, S., Tanenbaum, D.M., Meyerson, M., et al. (2004). Loss of heterozygosity and its correlation with expression profiles in subclasses of invasive breast cancers. Cancer Res. 64, 64-71.
Wilkie, S., Van Schalkwyk, M.C.I., Hobbs, S., Davies, D.M., Van, D.S., Pereira, A.C.P., Burbridge, S.E., Box, C., Eccles, S.A., and Maher, J. (2012). Dual targeting of ErbB2 and MUC1 in breast cancer using chimeric antigen receptors engineered to provide complementary signaling. J. Clin. Immunol. 32, 1059-1070.
Wu, C.-Y., Roybal, K.T., Puchner, E.M., Onuffer, J., and Lim, W.A. (2015). Remote control of therapeutic T cells through a small molecule-gated chimeric receptor. Science (80-. ). 350, aab4077.
Yeung, J.T., Hamilton, R.L., Ohnishi, K., Ikeura, M., Potter, D.M., Nikiforova, M.N., Ferrone, S., Jakacki, R.I., Pollack, I.F., and Okada, H. (2013). LOH in the HLA class I region at 6p21 is associated with shorter survival in newly diagnosed adult glioblastoma. Clin. Cancer Res. 19, 1816-1826.

## Claims

1. A safe effector immune cell for use in treating cancer in a patient having a tumor **characterized by** LOH of a single allelic variant encoding a polymorphic cell surface epitope,
wherein the cell is transfected with a nucleic acid molecule comprising a nucleotide sequence encoding an inhibitory chimeric antigen receptor (iCAR) capable of preventing or attenuating undesired activation of an effector immune cell; and an intracellular domain comprising at least one signal transduction element that inhibits an effector immune cell,
wherein the iCAR is directed to a single allelic variant encoding a polymorphic cell surface epitope absent from cells of the tumor due to loss of heterozygosity (LOH) but present at least on all cells of related mammalian normal tissue of the patient,
and wherein the polymorphic cell surface epitope is an HLA type I.

2. The safe effector immune cell for use according to claim 1, wherein the polymorphic cell surface epitope is HLA-A, HLA-B or HLA-C.

3. The safe effector immune cell for use according to claim 1, wherein said inhibitory chimeric antigen receptor comprises an extracellular domain comprising (i) an antibody, derivative or fragment thereof, such as a humanized antibody; a human antibody; a functional fragment of an antibody; a single-domain antibody, such as a Nanobody; a recombinant antibody; and a single chain variable fragment (ScFv); (ii) an antibody mimetic, such as an affibody molecule; an affilin; an affimer; an affitin; an alphabody; an anticalin; an avimer; a DARPin; a fynomer; a Kunitz domain peptide; and a monobody; or (iii) an aptamer.

4. The safe effector immune cell for use according to claim 1, wherein said mammalian tissue is human tissue and said related mammalian normal tissue is normal tissue from which the tumor developed.

5. The safe effector immune cell for use according to claim 1, wherein said at least one signal transduction element capable of inhibiting an effector immune cell is homologous to a signal transduction element of an immune checkpoint protein.

6. The safe effector immune cell for use according to claim 5, wherein said immune checkpoint protein is selected from the group consisting of PD1; CTLA4; BTLA; 2B4; CD160; CEACAM, such as CEACAM1; KIRs, such as KIR2DL1, KIR2DL2, KIR2DL3, KIR2DL4, KIR2DL5A, KIR2DL5B, KIR3DL1, KIR3DL2, KIR3DL3, LIR1, LIR2, LIR3, LIR5, LIR8 and CD94-NKG2A; LAG3; TIM3; V-domain Ig suppressor of T cell activation (VISTA); STimulator of INterferon Genes (STING); immunoreceptor tyrosine-based inhibitory motif (ITIM)-containing proteins, T cell immunoglobulin and ITIM domain (TIGIT), and adenosine receptor (e.g. A2aR).

7. The safe effector immune cell for use according to claim 1, wherein said extracellular domain is fused through a flexible hinge and transmembrane canonic motif to said intracellular domain.

8. The safe effector immune cell for use according to any one of claim 1 to 7, wherein the cell is transfected with a nucleic acid molecule comprising a nucleic acid molecule comprising a nucleotide sequence encoding an aCAR comprising an extracellular domain specifically binding a non-polymorphic cell surface epitope of an antigen or a single allelic variant of a polymorphic cell surface epitope, wherein said epitope is a tumor-associated antigen or is shared at least by cells of related tumor and normal tissue, and an intracellular domain comprising at least one signal transduction element that activates and/or co-stimulates an effector immune cell, optionally wherein
a) the extracellular domain of the aCAR specifically binds to a non-polymorphic cell surface epitope of an antigen and the extracellular domain of the iCAR specifically binds a single allelic variant of a polymorphic cell surface epitope of a different antigen than that to which the extracellular domain of said aCAR binds, and / or
b) wherein the extracellular domain of the aCAR specifically binds to a non-polymorphic cell surface epitope selected from the antigens listed in Table 1, such as CD 19.

9. The safe effector immune cell for the use of any one of claims 1 to 8, expressing on its surface an aCAR comprising an extracellular domain that specifically binds to a tumor-associated antigen or a non-polymorphic cell surface epitope of an antigen and an iCAR comprising an extracellular domain that specifically binds a single allelic variant of a polymorphic cell surface epitope of an antigen expressed at least in a tissue of origin of the tumor or of a housekeeping protein, such as an HLA-A, which is a different antigen than that to which the extracellular domain of said aCAR binds.

10. The safe effector immune cell for the use of any one of claims 1 to 8, which is an autologous or a universal (allogeneic) effector cell.

11. The safe effector immune cell for the use of any one of claims 1 to 10, selected from a T cell, natural killer cell or cytokine-induced killer cell.

## Patentansprüche

1. Sichere Effektor-Immunzelle zur Verwendung beim Behandeln von Krebs bei einem Patienten, der einen Tumor hat, der durch LOH einer einzelnen allelischen Variante gekennzeichnet ist, die für ein polymorphes Zelloberflächenepitop kodiert,
wobei die Zelle mit einem Nukleinsäuremolekül transfiziert ist, umfassend eine Nukleotidsequenz, die für einen inhibitorischen chimären Antigenrezeptor (iCAR) kodiert, der in der Lage ist, eine unerwünschte Aktivierung einer Effektor-Immunzelle zu verhindern oder abzuschwächen; und eine intrazelluläre Domäne, umfassend mindestens ein Signaltransduktionselement, das eine Effektor-Immunzelle inhibiert,
wobei der iCAR auf eine einzelne allelische Variante gerichtet ist, die für ein polymorphes Zelloberflächenepitop kodiert, das aufgrund eines Verlusts von Heterozygotie (LOH) in den Zellen des Tumors fehlt, aber zumindest an allen Zellen von verwandtem normalem Säugetiergewebe des Patienten vorhanden ist,
und wobei das polymorphe Zelloberflächenepitop ein HLA-Typ I ist.

2. Sichere Effektor-Immunzelle zur Verwendung nach Anspruch 1, wobei das polymorphe Zelloberflächenepitop HLA-A, HLA-B oder HLA-C ist.

3. Sichere Effektor-Immunzelle zur Verwendung nach Anspruch 1, wobei der inhibitorische chimäre Antigenrezeptor eine extrazelluläre Domäne umfasst, umfassend (i) einen Antikörper, ein Derivat oder Fragment davon, wie beispielsweise einen humanisierten Antikörper; einen humanen Antikörper; ein funktionelles Fragment eines Antikörpers; einen Einzeldomänenantikörper, wie beispielsweise einen Nanokörper; einen rekombinanten Antikörper; und ein variables Einzelkettenfragment (ScFv); (ii) ein Antikörpermimetikum, wie beispielsweise ein Affibody-Molekül; ein Affilin; ein Affimer; ein Affitin; ein Alphabody; ein Anticalin; ein Avimer; ein DARPin; ein Fynomer; ein Kunitz-Domänenpeptid; und einen Monokörper; oder (iii) ein Aptamer.

4. Sichere Effektor-Immunzelle zur Verwendung nach Anspruch 1, wobei das Säugetiergewebe humanes Gewebe ist und das verwandte normale Säugetiergewebe normales Gewebe ist, aus dem sich der Tumor entwickelt hat.

5. Sichere Effektor-Immunzelle zur Verwendung nach Anspruch 1, wobei das mindestens eine Signaltransduktionselement, das in der Lage ist, eine Effektor-Immunzelle zu inhibieren, homolog zu einem Signaltransduktionselement eines Immuncheckpoint-Proteins ist.

6. Sichere Effektor-Immunzelle zur Verwendung nach Anspruch 5, wobei das ImmunCheckpoint-Protein ausgewählt ist aus der Gruppe, bestehend aus PD1; CTLA4; BTLA; 2B4; CD160; CEACAM, wie beispielsweise CEACAM1; KIRs, wie beispielsweise KIR2DL1, KIR2DL2, KIR2DL3, KIR2DL4, KIR2DL5A, KIR2DL5B, KIR3DL1, KIR3DL2, KIR3DL3, LIR1, LIR2, LIR3, LIR5, LIR8 und CD94-NKG2A; LAG3; TIM3; V-Domänen-Ig-Suppressor von T-Zell-Aktivierung (VISTA); STimulator von INterferon-Genen (STING); Proteinen, die Immunrezeptor-Tyrosin-basiertes inhibitorisches Motiv enthalten, T-Zell-Immunoglobulin und ITIM-Domaine (TIGIT), und Adenosin-Rezeptor (z. B. A2aR).

7. Sichere Effektor-Immunzelle zur Verwendung nach Anspruch 1, wobei die extrazelluläre Domäne über ein flexibles Gelenk und ein transmembranes kanonisches Motiv an die intrazellulären Domäne kondensiert ist.

8. Sichere Effektor-Immunzelle zur Verwendung nach einem der Ansprüche 1 bis 7, wobei die Zelle mit einem Nukleinsäuremolekül transfiziert ist, umfassend ein Nukleinsäuremolekül, umfassend eine Nukleotidsequenz, die für ein aCAR kodiert, umfassend eine extrazelluläre Domäne, die spezifisch ein nicht polymorphes Zelloberflächenepitop eines Antigens oder eine einzelne allelische Variante eines polymorphen Zelloberflächenepitops bindet, wobei das Epitop ein tumorassoziiertes Antigen ist oder zumindest von Zellen eines verwandten Tumors und normalem Gewebe geteilt wird, und eine intrazelluläre Domäne, umfassend mindestens ein Signaltransduktionselement, das eine Effektor-Immunzelle aktiviert und/oder mitstimuliert, optional wobei
a) die extrazelluläre Domäne des aCAR spezifisch an ein nicht-polymorphes Zelloberflächenepitop eines Antigens bindet und die extrazelluläre Domäne des iCAR spezifisch an eine einzige allelische Variante eines polymorphen Zelloberflächenepitops eines anderen Antigens bindet als das, an das die extrazelluläre Domäne des aCAR bindet, und / oder
b) wobei die extrazelluläre Domäne des aCAR spezifisch an ein nicht-polymorphes Zelloberflächenepitop bindet, das ausgewählt ist aus den in Tabelle 1 aufgeführten Antigenen, wie beispielsweise CD 19.

9. Sichere Effektor-Immunzelle zur Verwendung nach einem der Ansprüche 1 bis 8, die auf ihrer Oberfläche einen aCAR exprimiert, umfassend eine extrazelluläre Domäne, die spezifisch an ein tumorassoziiertes Antigen oder ein nicht-polymorphes Zelloberflächenepitop eines Antigens bindet, und einen iCAR, umfassend eine extrazelluläre Domäne, die spezifisch eine einzelne allelische Variante eines polymorphen Zelloberflächenepitops eines Antigens, das zumindest in einem Ursprungsgewebe des Tumors exprimiert wird, oder eines Housekeeping-Proteins bindet, wie beispielsweise ein HLA-A, das ein anderes Antigen ist als das, an das die extrazelluläre Domäne des aCAR bindet.

10. Sichere Effektor-Immunzelle zur Verwendung nach einem der Ansprüche 1 bis 8, die eine autologe oder eine universelle (allogene) Effektorzelle ist.

11. Sichere Effektor-Immunzelle zur Verwendung nach einem der Ansprüche 1 bis 10, ausgewählt aus einer T-Zelle, natürlichen Killerzelle oder zytokininduzierten Killerzelle.

## Revendications

1. Cellule immunitaire effectrice sûre destinée à être utilisée dans le traitement du cancer chez un patient présentant une tumeur **caractérisée par** une LOH d'une variante allélique unique codant pour un épitope de surface cellulaire polymorphe,
dans laquelle la cellule est transfectée avec une molécule d'acide nucléique comprenant une séquence nucléotidique codant pour un récepteur d'antigène chimérique inhibiteur (iCAR) capable de prévenir ou d'atténuer l'activation indésirable d'une cellule immunitaire effectrice ; et un domaine intracellulaire comprenant au moins un élément de transduction du signal qui inhibe une cellule immunitaire effectrice,
dans laquelle l'iCAR est dirigé vers une variante allélique unique codant pour un épitope de surface cellulaire polymorphe absent des cellules de la tumeur en raison d'une perte d'hétérozygotie (LOH), mais présent au moins sur toutes les cellules du tissu mammalien normal apparenté du patient,
et dans laquelle épitope de surface cellulaire polymorphe est un HLA de type I.

2. Cellule immunitaire effectrice sûre destinée à être utilisée selon la revendication 1, dans laquelle épitope de surface cellulaire polymorphe est HLA-A, HLA-B ou HLA-C.

3. Cellule immunitaire effectrice sûre destinée à être utilisée selon la revendication 1, dans laquelle ledit récepteur d'antigène chimérique inhibiteur comprend un domaine extracellulaire comprenant (i) un anticorps, un dérivé ou un fragment de celui-ci, tel qu'un anticorps humanisé ; un anticorps humain ; un fragment fonctionnel d'un anticorps ; un anticorps à domaine unique, tel qu'un nanocorps ; un anticorps recombinant ; et un fragment variable à chaîne unique (ScFv) ; (ii) un mimétique d'anticorps, tel qu'une molécule afficorps ; une affiline ; un affimère ; une affitine ; un alphabody ; une anticaline ; un avimère ; une DARPin ; un fynomère ; un peptide du domaine de Kunitz ; et un monocorps ; ou (iii) un aptamère.

4. Cellule immunitaire effectrice sûre destinée à être utilisée selon la revendication 1, dans laquelle ledit tissu mammalien est un tissu humain et ledit tissu normal mammalien apparenté est un tissu normal à partir duquel la tumeur s'est développée.

5. Cellule immunitaire effectrice sûre destinée à être utilisée selon la revendication 1, dans laquelle ledit au moins un élément de transduction du signal capable d'inhiber une cellule immunitaire effectrice est homologue à un élément de transduction du signal d'une protéine de point de contrôle immunitaire.

6. Cellule immunitaire effectrice sûre destinée à être utilisée selon la revendication 5, dans laquelle ladite protéine de point de contrôle immunitaire est choisie dans le groupe constitué par PD 1 ; CTLA4 ; BTLA ; 2B4 ; CD 160 ; CEACAM, tel que CEACAM1 ; KIR, tel que KIR2DL1, KIR2DL2, KIR2DL3, KIR2DL4, KIR2DL5A, KIR2DL5B, KIR3DL1, KIR3DL2, KIR3DL3, LIR1, LIR2, LIR3, LIR5, LIR8 et CD94-NKG2A ; LAG3 ; TIM3 ; Ig à domaine V supprimant l'activation des cellules T (VISTA) ; stimulateur des gènes de l'interféron (STING) ; motif inhibiteur basé sur la tyrosine des immunorécepteurs (ITIM) - contenant des protéines, l'immunoglobuline des cellules T et le domaine ITIM (TIGIT), et un récepteur de l'adénosine (par ex. A2aR).

7. Cellule immunitaire effectrice sûre destinée à être utilisée selon la revendication 1, dans laquelle le domaine extracellulaire est fusionné au domaine intracellulaire par une charnière flexible et un motif canonique transmembranaire.

8. Cellule immunitaire effectrice sûre destinée à être utilisée selon l'une quelconque des revendications 1 à 7, dans laquelle la cellule est transfectée avec une molécule d'acide nucléique comprenant une molécule d'acide nucléique comprenant une séquence nucléotidique codant pour un aCAR comprenant un domaine extracellulaire liant spécifiquement un épitope de surface cellulaire non polymorphe d'un antigène ou une variante allélique unique d'un épitope de surface cellulaire polymorphe, dans laquelle ledit épitope est un antigène associé à une tumeur ou est partagé au moins par des cellules d'une tumeur apparentée et d'un tissu normal, et un domaine intracellulaire comprenant au moins un élément de transduction du signal qui active et/ou co-stimule une cellule immunitaire effectrice, éventuellement dans laquelle
a) le domaine extracellulaire de l'aCAR se lie spécifiquement à un épitope de surface cellulaire non polymorphe d'un antigène et le domaine extracellulaire de l'iCAR se lie spécifiquement à une variante allélique unique d'un épitope de surface cellulaire polymorphe d'un antigène différent de celui auquel se lie le domaine extracellulaire de l'aCAR, et / ou
b) dans laquelle le domaine extracellulaire de l'aCAR se lie spécifiquement à un épitope de surface cellulaire non polymorphe choisi parmi les antigènes énumérés dans le tableau 1, tels que CD 19.

9. Cellule immunitaire effectrice sûre destinée à être utilisée selon l'une quelconque des revendications 1 à 8, exprimant à sa surface un aCAR comprenant un domaine extracellulaire qui se lie spécifiquement à un antigène associé à la tumeur ou à un épitope de surface cellulaire non polymorphe d'un antigène et un iCAR comprenant un domaine extracellulaire qui se lie spécifiquement à une variante allélique unique d'un épitope de surface cellulaire polymorphe d'un antigène exprimé au moins dans un tissu d'origine de la tumeur ou d'une protéine d'entretien, telle qu'une HLA-A, qui est un antigène différent de celui auquel se lie le domaine extracellulaire dudit aCAR.

10. Cellule immunitaire effectrice sûre destinée à être utilisée selon l'une quelconque des revendications 1 à 8, qui est une cellule effectrice autologue ou universelle (allogène).

11. Cellule immunitaire effectrice sûre destinée à être utilisée selon l'une quelconque des revendications 1 à 10, choisie parmi une cellule T, une cellule tueuse naturelle ou une cellule tueuse induite par une cytokine.
